# EUROPEAN PATENT SPECIFICATION

(11) **EP 3 741 718 B1**
(45) Date of publication and mention of the grant of the patent: **01.03.2023**
(21) Application number: 19771563.4
(22) Date of filing: 12.03.2019
(51) Int. Cl.: B65H 19/16, A61F 13/15, B65H 23/02, B65H 19/18

(54) **ABSORBENT ARTICLE MANUFACTURING METHOD AND ABSORBENT ARTICLE MANUFACTURING DEVICE**
VERFAHREN ZUR HERSTELLUNG EINES SAUGFÄHIGEN ARTIKELS UND VORRICHTUNG ZUR HERSTELLUNG EINES SÄUGFÄHIGEN ARTIKELS
PROCÉDÉ DE FABRICATION D'ARTICLE ABSORBANT ET DISPOSITIF DE FABRICATION D'ARTICLE ABSORBANT

(30) Priority: 20.03.2018 JP 2018052123
(43) Date of publication of application: 25.11.2020
(73) Proprietor: Unicharm Corporation, Ehime 799-0111 (JP)
(72) Inventor: ISHIKAWA, Yoshihide, Kanonji-shi, Kagawa 769-1602 (JP); TADA, Hiroaki, Kanonji-shi, Kagawa 769-1602 (JP); OOHIRO, Takakazu, Kanonji-shi, Kagawa 769-1602 (JP)
(74) Representative: Staeger & Sperling Partnerschaftsgesellschaft mbB
(86) International application number: PCT/JP2019/009983
(87) International publication number: WO 2019/181648

(56) References cited:
- WO-A1-2017/149610
- JP-A- H02 188 349
- JP-A- H09 183 542
- US-A- 4 543 152

## Description

### [Technical Field]

The present invention relates to a method and a device for manufacturing an absorbent article.

### [Background Art]

The following method for manufacturing an absorbent article is well-known: without stopping the feeding of the preceding material, a following material supplied from a following-material coil is joined to a preceding material that is continuously fed from a preceding-material coil, then feeding the following material (e.g., see Patent Document 1).

### [Citation List]

### [Patent Literature]

[Patent Document 1] Japanese Patent Application Publication No. H2-188349. Further prior art in this technical field is disclosed in document US 4,543,152 A and in document WO2017/149610A1.

### [Summary of Invention]

### [Technical Problem]

However, the preceding material and the following material are sometimes out of alignment in the width direction when joining is to be performed, and in such a case, the preceding material and the following material are joined out of alignment in the width direction, thus leading to problems such as large swerving of the conveyed material or separation of the materials during conveying due to insufficient joining strength.

The present invention was achieved in light of conventional problems such as that described above, and an aspect of the present invention is to suppress misalignment when the preceding material and the following material are joined.

### [Solution to Problem]

A main aspect of the present invention for achieving the above-described aspect is a method as defined in claim 1 for manufacturing an absorbent article in which, without stopping feeding of a first material that has been continuously fed from a preceding first material coil, a second material supplied from a following second material coil is joined to the first material, then feeding the second material,
the method for manufacturing the absorbent article including:
   a first-material-coil attaching step of attaching the first material coil to a first rotation shaft, the first material being wound around the first material coil;
   a second-material-coil attaching step of attaching a second material coil to a second rotation shaft, the second material being wound around the second material coil; and
   a joining step of joining the second material to the first material by pressing the first material against an outer circumferential surface of the second material coil,
      the first material being fed from the first material coil due to driven rotation of the first rotation shaft,
      the second material coil rotating due to driven rotation of the second rotation shaft,
   a size of the first material coil in a diameter direction being larger than a size of the first material coil in a width direction,
   a size of the second material coil in a diameter direction being larger than a size of the second material coil in a width direction,
in the first-material-coil attaching step,
   the first material coil being attached while being sandwiched from two widthwise sides of the first material coil between a first positioning portion and a first deformation-suppressing member,
   the first positioning portion being provided at a predetermined position in an axial direction of the first rotation shaft
   the first deformation-suppressing member being a member for suppressing widthwise deformation of the first material coil,
in the second-material-coil attaching step,
   the second material coil being attached while being sandwiched from two widthwise sides of the second material coil between a second positioning portion and a second deformation-suppressing member,
   the second positioning portion being provided at a predetermined position in an axial direction of the second rotation shaft,
   the second deformation-suppressing member being a member for suppressing widthwise deformation of the second material coil.

Features of the present invention other than the above will become clear by reading the description of the present specification with reference to the accompanying drawings.

### [Advantageous Effects of Invention]

According to the present invention, it is possible to suppress misalignment when the preceding material and the following material are joined.

### [Brief Description of the Drawings]

FIG. 1 is a schematic view of a manufacturing line of an absorbent-article manufacturing device 1.
FIG. 2A is a front schematic view of a material supply device 10 that is feeding a preceding material 3ps from a preceding-material coil 3p, and the view indicated by arrows A-A is a lateral schematic view of the preceding-material coil 3p.
FIG. 2B is a front schematic view of the material supply device 10 in a state where a following-material coil 3f has been attached, and the view indicated by arrows B-B is a lateral schematic view of the following-material coil 3f.
FIG. 2C is a front schematic view of the material supply device 10 in a state where a turret rotation portion 20 is turning.
FIG. 2D is a front schematic view of the material supply device 10 in a state where a following material 3fs is being joined to the preceding material 3ps.
In FIG. 3, the upper portion is a top schematic view of a correction device 50 in a view along arrows C-C in FIG. 2B, and the lower portion of FIG. 3 is a front schematic view of the correction device 50 in a view along arrows D-D in FIG. 2B.
FIG. 4 is a diagram showing a relationship between a control unit 100 and other devices.
FIG. 5 is a flowchart showing control of operations of the material supply device 10.
FIG. 6 is a cross-sectional schematic view of a first positioning portion 25pa and a first pressing plate 25pb that are attached to a first rotation shaft 25p, in a view along arrows E-E in FIG. 2A.

### [Description of Embodiments]

At least the following matters will become clear with the description of this specification and the attached drawings. A method for manufacturing an absorbent article in which, without stopping feeding of a first material that has been continuously fed from a preceding first material coil, a second material supplied from a following second material coil is joined to the first material, then feeding the second material,
the method for manufacturing the absorbent article including:
a first-material-coil attaching step of attaching the first material coil to a first rotation shaft, the first material being wound around the first material coil;
a second-material-coil attaching step of attaching a second material coil to a second rotation shaft, the second material being wound around the second material coil; and
a joining step of joining the second material to the first material by pressing the first material against an outer circumferential surface of the second material coil,
   the first material being fed from the first material coil due to driven rotation of the first rotation shaft,
   the second material coil rotating due to driven rotation of the second rotation shaft,
a size of the first material coil in a diameter direction being larger than a size of the first material coil in a width direction,
a size of the second material coil in a diameter direction being larger than a size of the second material coil in a width direction,
in the first-material-coil attaching step,
   the first material coil being attached while being sandwiched from two widthwise sides of the first material coil between a first positioning portion and a first deformation-suppressing member,
   the first positioning portion being provided at a predetermined position in an axial direction of the first rotation shaft
   the first deformation-suppressing member being a member for suppressing widthwise deformation of the first material coil,
in the second-material-coil attaching step,
   the second material coil being attached while being sandwiched from two widthwise sides of the second material coil between a second positioning portion and a second deformation-suppressing member,
   the second positioning portion being provided at a predetermined position in an axial direction of the second rotation shaft,
   the second deformation-suppressing member being a member for suppressing widthwise deformation of the second material coil.

According to this method for manufacturing an absorbent article, it is possible to suppress misalignment when the preceding material and the following material are joined.

In such a method for manufacturing an absorbent article, it is desirable
that the first deformation-suppressing member is a first pressing plate that has a predetermined length in a diameter direction of the first rotation shaft, and
that the predetermined length of the first pressing plate is smaller than a radius of the first material coil before being fed as the first material.

According to this method for manufacturing an absorbent article, it is possible to suppress the case where the belt member comes into contact with the first pressing plate when the preceding material and the following material are joined.

In such a method for manufacturing an absorbent article, it is desirable
that the first deformation-suppressing member is a first pressing plate that has a predetermined length in a diameter direction of the first rotation shaft, and
that the predetermined length of the first pressing plate is larger than 113 of a radius of the first material coil before being fed as the first material.

According to this method for manufacturing an absorbent article, it is possible to further suppress misalignment of the material coil in the width direction.

In such a method for manufacturing an absorbent article, it is desirable
that the second deformation-suppressing member is a second pressing plate that has a predetermined length in a diameter direction of the second rotation shaft, and
that the predetermined length of the second pressing plate is smaller than a radius of the second material coil before being fed as the second material.

According to this method for manufacturing an absorbent article, it is possible to suppress the case where the belt member comes into contact with the second pressing plate when the preceding material and the following material are joined.

In such a method for manufacturing an absorbent article, it is according to the invention
that the second deformation-suppressing member is a second pressing plate that has a predetermined length in a diameter direction of the second rotation shaft, and
that the predetermined length of the second pressing plate is larger than 113 of a radius of the second material coil before being fed as the second material.

According to this method for manufacturing an absorbent article, it is possible to further suppress misalignment of the material coil in the width direction.

In such a method for manufacturing an absorbent article, it is desirable
that the first positioning portion is plate-shaped and has a predetermined length in a diameter direction of the first rotation shaft, and
that the predetermined length of the first positioning portion is smaller than a radius of the first material coil before being fed as the first material.

According to this method for manufacturing an absorbent article, it is possible to suppress the case where the belt member comes into contact with the first positioning portion when the preceding material and the following material are joined.

In such a method for manufacturing an absorbent article, it is according to the invention
that the second positioning portion is plate-shaped and has a predetermined length in a diameter direction of the second rotation shaft, and
that the predetermined length of the second positioning portion is smaller than a radius of the second material coil before being fed as the second material.

According to this method for manufacturing an absorbent article, it is possible to suppress the case where the belt member comes into contact with the second positioning portion when the preceding material and the following material are joined.

In such a method for manufacturing an absorbent article, it is desirable that
the method further comprises a correcting step of correcting a position of the outer circumferential surface of the second material coil in an axial direction of the second rotation shaft,
the correcting being performed such that a feeding position of the first material in an axial direction of the first rotation shaft matches the position of the outer circumferential surface of the second material coil in the axial direction of the second rotation shaft,
the correcting being performed by a correction device configured to perform correction by coming into contact with a side surface of an outer circumferential portion of the second material coil attached to the second rotation shaft and moving the outer circumferential portion from one side toward another side in the axial direction of the second rotation shaft.

According to this method for manufacturing an absorbent article, it is possible to further suppress misalignment when the preceding material and the following material are joined.

In such a method for manufacturing an absorbent article, it is desirable
that at least one of the correction device is provided for the second rotation shaft,
that the correction device includes a moving portion configured to move along a diameter direction of the second rotation shaft,
that in the second-material-coil attaching step,
   the correction device is retracted to a retracted position so as not to come into contact with the second material coil that is to be attached,
   the retracted position being a position that is outward in the diameter direction with respect to the outer circumferential surface of the second material coil that is to be attached, and
that in the correcting step,
   the correction device is moved inward in the diameter direction of the second rotation shaft from the retracted position so as to come into contact with the side surface of the outer circumferential portion of the second material coil.

According to this method for manufacturing an absorbent article, it is possible to suppress the case where the following-material coil comes into contact with the correction device when the following-material coil is attached.

In such a method for manufacturing an absorbent article, it is desirable that
a contact portion of the correction device that comes into contact with the second material coil is constituted by a rotating body that can rotate about a rotation axis extending along a diameter direction of the second rotation shaft.

According to this method for manufacturing an absorbent article, compared to the case where the contact portion cannot rotate, it is possible to suppress frictional force between the contact portion and the following-material coil.

In such a method for manufacturing an absorbent article, it is desirable that
the rotating body has a tapered shape according to which a diameter of the rotating body increases from an inward side toward an outward side in the diameter direction of the second rotation shaft.

According to this method for manufacturing an absorbent article, the correction can be made a larger amount outside than inside in the outer circumferential direction of the following-material coil.

In such a method for manufacturing an absorbent article, it is desirable that
in the correcting step,
the correction device performs correction over an entire circumference of the outer circumferential portion of the second material coil using at least either one of
a method in which the second material coil is rotated around the second rotation shaft in a state where the correction device is fixed at a predetermined position in a circumferential direction of the second material coil, and
a method in which a rotation moving portion is provided for movement of the correction device along the circumferential direction of the second material coil centered about the second rotation shaft, and in which the correction device is moved along the circumferential direction of the second material coil by the rotation moving portion in a state where rotation of the second material coil is temporarily stopped.

According to this method for manufacturing an absorbent article, correction can be performed over the entire circumference of the following-material coil.

In such a method for manufacturing an absorbent article, it is desirable
that the method further comprises a detecting step of detecting the position of the outer circumferential surface of the second material coil in the axial direction of the second rotation shaft with use of a detection portion, and
that in the correcting step,
   a correction direction and a correction amount of the correction device are controlled based on information indicating an amount of deviation between
   the feeding position of the first material in the axial direction of the first rotation shaft and
   the position of the outer circumferential surface of the second material coil in the axial direction of the second rotation shaft, the position having been detected in the detecting step.

According to this method for manufacturing an absorbent article, it is possible to further suppress misalignment when the preceding material and the following material are joined.

In such a method for manufacturing an absorbent article, it is desirable
that a winding end portion of the second material is located at the outer circumferential surface of the second material coil, and
that the winding end portion is fixed to the second material coil using a joining member so at to be prevented from separating from the second material coil.

According to this method for manufacturing an absorbent article, it is possible to suppress misalignment of the winding end portion in the width direction.

In such a method for manufacturing an absorbent article, it is desirable
that in the first-material-coil attaching step,
   an attachment robot attaches the first material coil to the first rotation shaft so as to abut against the first positioning portion, and
   then after the attachment, the attachment robot attaches the first deformation-suppressing member to the first rotation shaft such that the first material coil is sandwiched from two widthwise sides of the first material coil by the first positioning portion and the first deformation-suppressing member, and
that in the second-material-coil attaching step,
   the attachment robot attaches the second material coil to the second rotation shaft so as to abut against the second positioning portion, and
   then after the attachment, the attachment robot attaches the second deformation-suppressing member to the second rotation shaft such that the second material coil is sandwiched from two widthwise sides of the second material coil by the second positioning portion and the second deformation-suppressing member.

According to this method for manufacturing an absorbent article, the material coil can be sandwiched between the positioning portion and the pressing plate and attached more quickly than in the case where such operations are performed manually.

A device for manufacturing an absorbent article according to the invention is defined in claim 14, including:
a first material coil around which a first material is wound,
   a size of the first material coil in a diameter direction being larger than a size of the first material coil in a width direction;
a second material coil around which a second material is wound,
   a size of the second material coil in a diameter direction being larger than a size of the second material coil in a width direction,
   without stopping feeding of the first material that has been continuously fed from the preceding first material coil, the second material supplied from the following second material coil being joined
to the first material and then being fed;
a first rotation shaft to which a first material coil is to be attached;
a connecting portion configured to join the second material to the first material by, through driven rotation of the first rotation shaft, pressing the first material fed from the first material coil against an outer circumferential surface of the second material coil that is rotating due to driven rotation of the second rotation shaft,
a first positioning portion that is provided at a predetermined position in an axial direction of the first rotation shaft;
a first deformation-suppressing member configured to suppress widthwise deformation of the first material coil,
   the first positioning portion and the first deformation-suppressing member being provided in order to sandwich the first material coil from two widthwise sides of the first material coil during attachment;
a second positioning portion that is provided at a predetermined position in an axial direction of the second rotation shaft; and
a second deformation-suppressing member configured to suppress widthwise deformation of the second material coil,
   the second positioning portion and the second deformation-suppressing member being provided in order to sandwich the second material coil from two widthwise sides of the second material coil during attachment.

This absorbent article manufacturing device achieves effects similar to those of the method for manufacturing an absorbent article described above.

Method for manufacturing an absorbent article of present embodiment An method for manufacturing an absorbent article according to the present embodiment is used in the manufacturing of absorbent articles in a material supply device 10 of an absorbent-article manufacturing device 1. Specifically, the absorbent-article manufacturing method (an absorbent-article manufacturing device) of the present embodiment is a method (device) for manufacturing absorbent articles, and is a material supply method (material supply device 10).

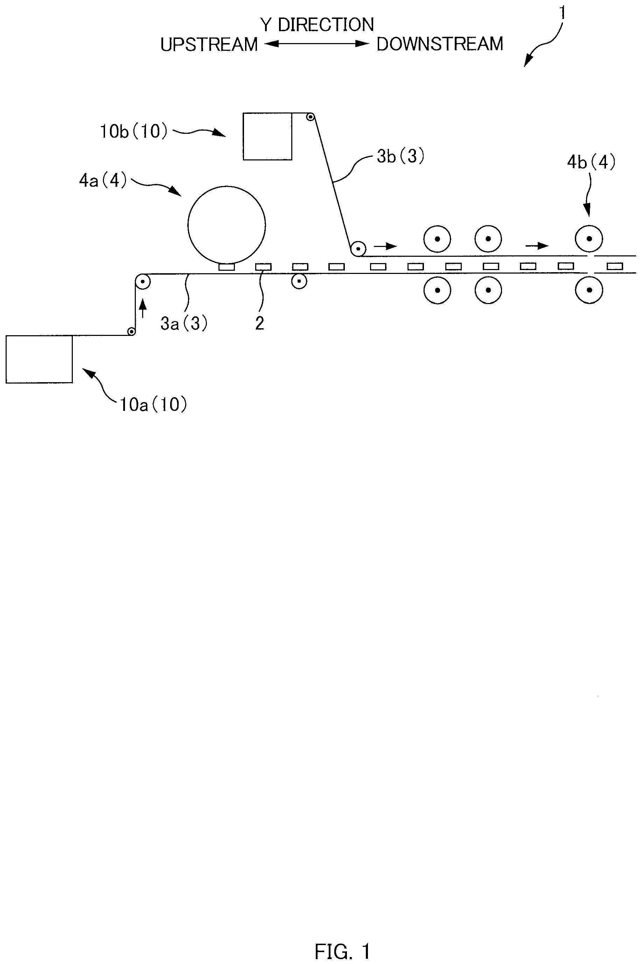

FIG. 1 is a schematic view of an example of a manufacturing line of the absorbent-article manufacturing device 1. Note that in order to make the description of the present invention easier to understand, several portions such as material supply devices 10 and processing units 4 are not shown in FIG. 1 (these omitted portions will also not be described below) . The lateral direction of the paper plane is the Y direction, and the left side (right side) of the paper plane is the upstream (downstream) side.

The absorbent-article manufacturing device 1 includes two material supply devices 10 (a first material supply device 10a and a second material supply device 10b) and two processing units 4 (an absorbent-body processing unit 4a and an end cutter 4b). Materials 3 are introduced to the first material supply device 10a and the second material supply device 10b in the form of material coils in which continuous sheets of the materials 3 are wound into coils. In other words, the first material supply device 10a and the second material supply device 10b feed continuous sheets of the materials 3 from material coils. Note that the first material supply device 10a and the second material supply device 10b are each provided for a corresponding type of material 3.

In the absorbent-article manufacturing device 1 of the present embodiment, the material 3a fed from the first material supply device 10a is conveyed downstream in the Y direction by a conveying mechanism (not shown), and when the installation position of the absorbent-body processing unit 4a on the conveying route is reached, an absorbent body 2 is attached (transferred) to the material 3a. The material 3a in this state is then further conveyed downstream in the Y direction by the conveying mechanism, the material 3b fed from the second material supply device 10b is arranged so as to sandwich the absorbent body 2 along with the material 3a, and then the attachment of various elastic strings, bonding, and the like are performed. The materials are then conveyed by the conveying mechanism to the installation position of the end cutter 4b on the conveying route, and the end cutter 4b cuts the materials into individual absorbent articles, thus producing completed absorbent articles.

Examples of the absorbent article include a sanitary napkin and a folded or pull-on disposable diaper, but the absorbent article may be any article that absorbs excreted fluid from the wearer.

Note that the absorbent article is constituted by various types of members such as the absorbent body 2 made of a superabsorbent polymer (SAP) or the like for absorbing excreted fluid, a top sheet that is made of liquid-permeable nonwoven fabric or the like and is arranged on the skin side of the absorbent body 2 in the thickness direction, a back sheet that is made of a liquid-impermeable film or the like and is arranged on the non-skin side of the absorbent body for preventing the leakage of excreted fluid to the non-skin side, barrier cuffs or leg gathers made of nonwoven fabric or the like for suppressing leakage around the legs, and elastic members such as elastic strings that allow the absorbent article to be put on. This configuration changes according to the application of the absorbent article, such as the various examples given above, and the processing units 4 and the material supply devices 10 provided in the absorbent-article manufacturing device 1 are also changed as appropriate for the application.

### Materials 3 and material coils

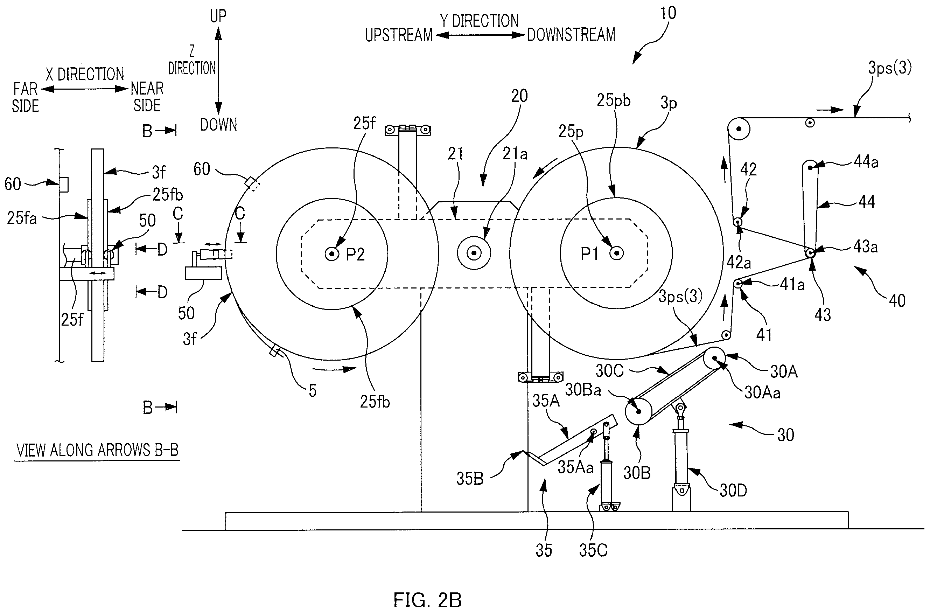

As previously described, the materials 3 of the present embodiment are continuous sheets that are fed from material coils in which the continuous materials 3 are wound. The material coils are each formed by attaching the winding start portion of one material 3 to a paper tube and winding the material 3 into a spiral, and then cutting the continuous sheet of material 3 when a predetermined outer diameter dimension (different for each type of material 3) is reached. Here, the winding end portion of the material 3 located on the outer circumferential surface of the material coil is fixed to the material coil using a joining member 5 so as to not become separated from the material coil (see FIGS. 2B to 2D) . The material 3, which forms the material coil that has a predetermined outer diameter dimension, is then transported from the material 3 manufacturing location to the absorbent-article manufacturing device 1, and then introduced to the material supply device 10 after transport.

Also, when the material 3 of the present embodiment is in the material coil form, the size of the material coil in the diameter direction is larger than the size in the width direction, and in the present embodiment, the size in the diameter direction is approximately 10 to 20 times larger than the size in the width direction. In other words, the size in the diameter direction of the material coil is sufficiently larger than the size in the width direction. Also, the material 3 of the present embodiment is a continuous sheet of flexible nonwoven fabric, such as air laid nonwoven fabric, spun lace nonwoven fabric, or air-through nonwoven fabric.

In other words, the material coil is constituted by highly flexible nonwoven fabric, and the size in the diameter direction thereof is sufficiently larger than the size in the width direction thereof. Accordingly, the material coil easily deforms in the width direction due to vibration during transport of the material coil or due to the weight of the material 3 when the width direction is pointed vertically upward when moving the material coil.

### Material supply device 10

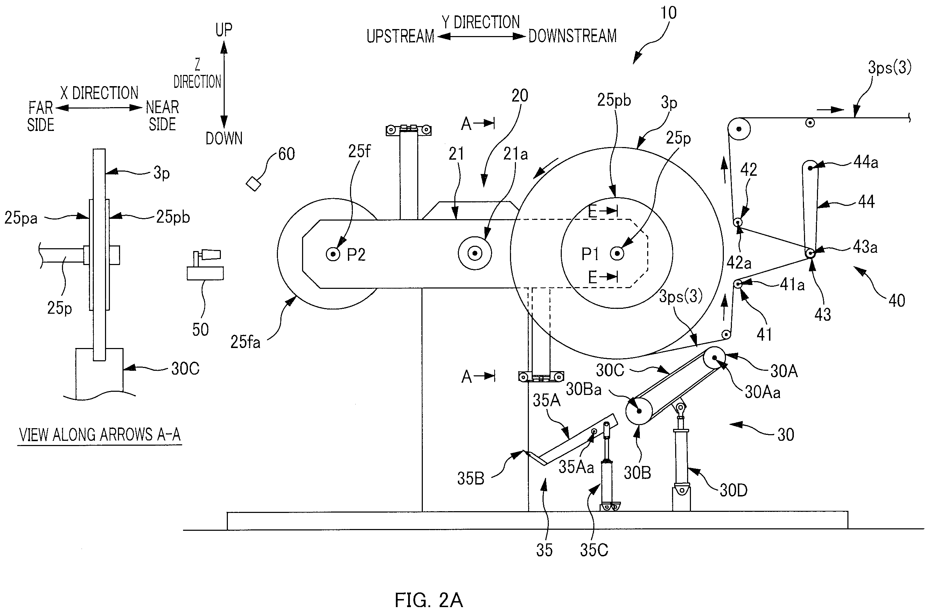

The following describes the material supply devices 10 with reference to FIGS. 2A and 2B. Note that although the first material supply device 10a and the second material supply device 10b are separately provided for the corresponding type of material 3 in the above described, the first material supply device 10a and the second material supply device 10b have the same basic configuration as each other in the present embodiment. Accordingly, the following describes one material supply device 10.

FIG. 2A is a front schematic view of the material supply device 10 in a state of feeding a preceding material 3ps (corresponding to a first material) from a preceding-material coil 3p (corresponding to a first material coil), and the view indicated by arrows A-A is a lateral schematic view of the preceding-material coil 3p. Note that in FIG. 2A, in order to facilitate understanding the present invention, the diameter of the preceding-material coil 3p is the diameter before the preceding material 3ps is fed. FIG. 2B is a front schematic view of the material supply device 10 in a state where a following-material coil 3f (corresponding to a second material coil) has been attached, and the view indicated by arrows B-B is a lateral schematic view of the following-material coil 3f. Note that in the figures of the present embodiment, members are sometimes omitted as appropriate from both the front schematic view and the lateral schematic view in order to facilitate understanding of the present invention. Also, an X direction, a Y direction, and a Z direction are three directions that are orthogonal to each other, and among these directions, the X direction and the Y direction are horizontal directions, and the Z direction is the vertical direction. In the front schematic view, letting the lateral direction of the paper surface be the Y direction, the left side (right side) of the paper surface is the upstream (downstream) side, and letting the vertical direction of the paper surface be the Z direction, the far side (near side) of the paper surface is the upper (lower) side, whereas in the lateral schematic view, letting the lateral direction of the paper surface be the X direction, the left side (right side) of the paper surface is the far (near) side.

Also, in the present embodiment, the positions of a first rotation shaft 25p and a second rotation shaft 25f are shown at a joining position P1 and an attachment position P2 in FIGS. 2A and 2B. Although described in detail later, when the first rotation shaft 25p is located at the joining position P1, the second rotation shaft 25f is located at the attachment position P2, and when the second rotation shaft 25f is located at the joining position P1, the first rotation shaft 25p is located at the attachment position P2.

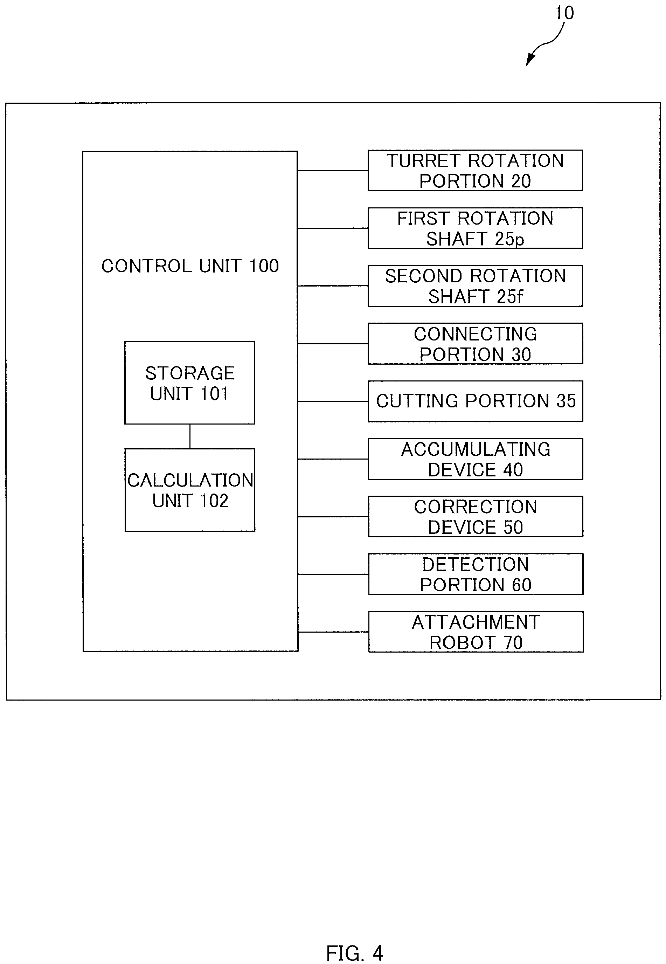

As shown in FIGS. 2A and 2B, the material supply device 10 includes: the first rotation shaft 25p that is located at the joining position P1; the second rotation shaft 25f that is located at the attachment position P2; a turret rotation portion 20 that switches the positions of the first rotation shaft 25p and the second rotation shaft 25f; a connecting portion 30 that joins the following material 3fs (corresponding to the second material) to the preceding material 3ps; a cutting portion 35 that cuts the preceding material 3ps after the following material 3fs has been joined to the preceding material 3ps; an accumulating device 40 that controls the tension (tensile force) of the material 3; a correction device 50 that corrects (adjusts) the position of the outer circumferential surface of the following-material coil 3f in the X direction; a detection portion 60 that detects the position of the outer circumferential surface of the following-material coil 3f in the X direction; an attachment robot 70 (see FIG. 4) that attaches, the attachment position P2, the preceding-material coil 3p and the following-material coil 3f to the first rotation shaft 25p or the second rotation shaft 25f at; and a control unit 100 (see FIG. 4) that controls the operations of various units.

The first rotation shaft 25p located at the joining position P1 includes: a motor (not shown) that is a motive power source for rotation about a rotation axis that extends in the X direction; and a first positioning portion 25pa that is an approximately circular plate-shaped member located at a predetermined position on the first rotation shaft 25p in the X direction. As shown by the A-A arrow view, the preceding-material coil 3p is attached on the near side of the first positioning portion 25pa in the X direction, and an approximately circular first pressing plate 25pb (corresponding to a first deformation-suppressing member) is attached on the near side of the preceding-material coil 3p in the X direction. The first positioning portion 25pa and the first pressing plate 25pb of the present embodiment have a smaller diameter than the preceding-material coil 3p before being fed, and the diameter of the first pressing plate 25pb is greater than 1/3 of the diameter of the preceding-material coil 3p before being fed. Specifically, the first deformation-suppressing member is the first pressing plate 25pb that has a predetermined length in the diameter direction of the first rotation shaft 25p, and the predetermined length of the first pressing plate 25pb is smaller than the radius of the first material coil (the preceding-material coil 3p) before being fed as the first material (the preceding material 3ps), and furthermore larger than 1/3 of that radius. Also, the first positioning portion 25pa is a plate-shaped member having a predetermined length in the diameter direction of the first rotation shaft 25p, and the predetermined length of the first positioning portion 25pa is smaller than the radius of the first material coil (the preceding-material coil 3p) before being fed as the first material (the preceding material 3ps).

Also, when the motor of the first rotation shaft 25p is driven to rotate, the preceding-material coil 3p rotates counter-clockwise, and the preceding material 3ps is fed downstream in the Y direction from the lower side of the preceding-material coil 3p in the Z direction. The fed preceding material 3ps passes through the accumulating device 40 and is supplied from the material supply device 10 to the conveying mechanism.

The second rotation shaft 25f located at the attachment position P2 includes : a motor (not shown) that is a motive power source for rotation about a rotation axis that extends in the X direction; and a second positioning portion 25fa that is an approximately circular plate-shaped member located at a predetermined position in the X direction. As shown by the B-B arrow view, the following-material coil 3f is attached on the near side of the second positioning portion 25fa in the X direction, and a second pressing plate 25fb (corresponding to a second deformation-suppressing member) is attached on the near side of the following-material coil 3f in the X direction. The second positioning portion 25fa and the second pressing plate 25fb of the present embodiment have a smaller diameter than the following-material coil 3f before being fed, and the diameter of the second pressing plate 25fb is greater than 1/3 of the diameter of the following-material coil 3f before being fed. Specifically, the second deformation-suppressing member is the second pressing plate 25fb that has a predetermined length in the diameter direction of the second rotation shaft 25f, and the predetermined length of the second pressing plate 25fb is smaller than the radius of the second material coil (the following-material coil 3f) before being fed as the second material (the following material 3fs), and furthermore larger than 1/3 of that radius. Also, the second positioning portion 25fa is a plate-shaped member having a predetermined length in the diameter direction of the second rotation shaft 25f, and the predetermined length of the second positioning portion 25fa is smaller than the radius of the second material coil (the following-material coil 3f) before being fed as the second material (the following material 3fs).

The turret rotation portion 20 is provided with an elongated plate-shaped turret 21. The turret 21 is supported to a turret shaft 21a including a motor (not shown) that is a motive power source for rotation about a rotation axis that extends in the X direction. The first rotation shaft 25p and the second rotation shaft 25f are provided on the turret 21 at positions that are in point symmetry about the turret shaft 21a. Accordingly, when the turret 21 rotates about the turret shaft 21a, the positions of the first rotation shaft 25p and the second rotation shaft 25f (i.e., the joining position P1 and the attachment position P2) can be switched.

The connecting portion 30 is provided below, in the Z direction, the preceding material 3ps that is fed at the joining position P1, and is a device for joining the following material 3fs to the preceding material 3ps. The connecting portion 30 includes: a first rotation roller 30A that rotates about a first fixed shaft 30Aa extending in the X direction; a swing arm that swings about the first fixed shaft 30Aa; a second rotation roller 30B that rotates about a second fixed shaft 30Ba provided at the end portion of the swing arm on the side opposite to the first fixed shaft 30Aa; a rotatable endless belt member 30C that is wound around the first rotation roller 30A and the second rotation roller 30B; a connecting-portion actuator 30D (e.g., an air cylinder) for swinging the belt member 30C (the swing arm); and a motor (not shown) serving as a motive power source for driving the first rotation roller 30A or the second rotation roller 30B to rotate.

In the connecting portion 30 of the present embodiment, the preceding material 3ps is pressed against the following-material coil 3f in order to join the preceding material 3ps to the following material 3fs (described in detail later in the section regarding operations of the material supply device 10). In order to perform this joining reliably, the X-direction size of the belt member 30C (including the first rotation roller 30A and the second rotation roller 30B that rotatably support the belt member 30C) that comes into contact with the preceding material 3ps during joining is larger than the X-direction size of the preceding material 3ps, as shown in the A-A arrow view. In other words, the width of the belt member 30C is larger than the width of the preceding material 3ps, the belt member 30C comes into contact with the entire width-direction range of the preceding material 3ps and is pressed against the entire width-direction range of the following-material coil 3f (having the same width as the preceding material 3ps) so as to join the following material 3fs to the preceding material 3ps.

The cutting portion 35 is provided at a location that is below, in the Z direction, the first material 3ps fed at the joining position P1 and that is upstream of the connecting portion 30 in the Y direction. The cutting portion 35 is a device for cutting the preceding material 3ps after the following material 3fs has been joined to the preceding material 3ps. The cutting portion 35 includes: an arm member 35A that is swingably supported to a rotation shaft 35Aa that extends in the X direction; a cutter blade 35B that is fixed to a swinging end portion of the arm member 35; and a cutting-portion actuator 35C (e.g., an air cylinder) that swings the arm member 35A.

The accumulating device 40 is a so-called dancer unit that accumulates the fed material 3 in the form of a loop that can be supplied to the conveying mechanism of the absorbent-article manufacturing device 1. The tension of the material 3 is controlled by adjusting the size of the loop, supplying the material 3 to the conveying mechanism of the absorbent-article manufacturing device 1.

The accumulating device 40 includes: an entrance roll 41 that is located at the entrance of the accumulating device 40 and is rotatably supported to a rotation shaft 41a that extends in the X direction; an exit roll 42 that is located at the exit of the accumulating device 40 and is rotatably supported to a rotation shaft 42a that extends in the X direction; a moving roll 43 that is guided so as to move back and forth in a predetermined direction (the Y direction in FIGS. 2A and 2B) for changing the size of the loop of the material 3 and is rotatably supported to a rotation shaft 43a that extends in the X direction; and an arm portion 44 that is swingably supported to a rotation shaft 44a that extends in the X direction in order to guide the moving roll 43 so as to be able to move back and forth in the predetermined direction. The material 3 is wound (wrapped) around the entrance roll 41, the moving roll 43, and the exit roll 42 in this order, thus forming the loop of the material 3.

The correction device 50 is a device that corrects the X-direction position of the outer circumferential surface of the following-material coil 3f that is attached at the attachment position P2. At least one correction device 50 is provided for the attachment position P2, and the correction device 50 is positioned so as to be able to be located radially outward with respect to the outer surface of the following-material coil 3f before being fed. As shown in FIG. 2B, in the present embodiment, one correction device 50 is provided at a 9 o' clock position relative to the attachment position P2 such that a later-described contact portion 51 comes into contact with the following-material coil 3f at the same position (same height) as the second rotation shaft 25f in the Z direction.

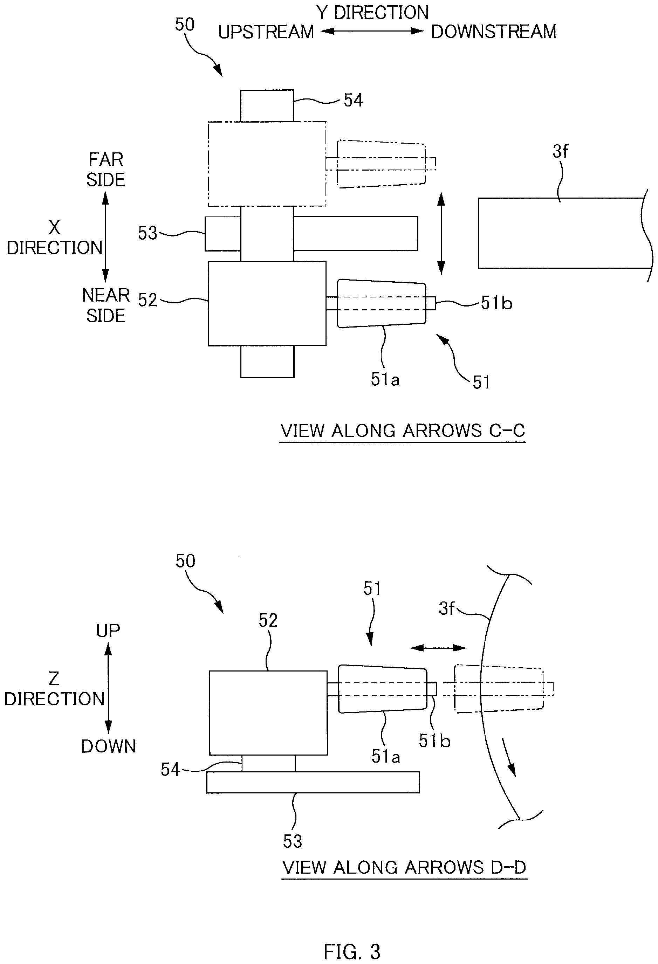

The upper portion of FIG. 3 is a top schematic view of the correction device 50 in a view along arrows C-C in FIG. 2B, and the lower portion of FIG. 3 is a front schematic view of the correction device 50 in a view along arrows D-D in FIG. 2B. Note that in both the top schematic view and the front schematic view, some members have been omitted as appropriate in order to facilitate understanding of the present invention.

As shown in the upper and lower portions of FIG. 3, the correction device 50 includes: a contact portion 51 that comes into contact with the outer circumferential portion of the following-material coil 3f; a contact-portion support portion 52 that supports the contact portion 51 so as to be capable of rotating about a rotation axis that extends in the Y direction; a first moving portion 53 that moves the contact-portion support portion 52 back and forth in the Y direction with use of a motor (not shown) ; and a second moving portion 54 that moves the contact-portion support portion 52 back and forth in the X direction with use of a motor (not shown) . In other words, at least one correction device 50 is provided for the second rotation shaft 25f, and includes a moving portion (the first moving portion 53) for movement along the diameter direction of the second rotation shaft 25f.

The contact portion 51 is constituted by a rotating body 51a and a rotating-body support portion 51b. The rotating-body support portion 51b is supported to the contact-portion support portion 52 such that the rotating body 51a can rotate about a rotation shaft that extends in the Y direction. Also, the rotating body 51a has a tapered shape in which the diameter increases from the inward side toward the outward side in the diameter direction of the second rotation shaft 25f (from the leading end of the rotating body 51a toward the terminal end) . In other words, the contact portion (contact portion 51) of the correction device 50 that comes into contact with the second material coil (the following-material coil 3f) is constituted by the rotating body 51a that can rotate about a rotation axis (the rotating-body support portion 51b) that extends in the diameter direction of the second rotation shaft 25f, and the rotating body 51a has a tapered shape in which the diameter of the rotating body 51a increases from the inward side toward the outward side in the diameter direction of the second rotation shaft 25f.

The detection portion 60 is a sensor that detects the position of the outer circumferential surface of the following-material coil 3f in the X direction before being fed. As shown in FIG. 2B, the detection portion 60 is provided at a position that is at approximately 10 o' clock relative to the attachment position P2 on the far side of the outer circumferential surface of the following-material coil 3f before being fed. The sensor serving as the detection portion 60 can be a laser displacement sensor, an ultrasonic displacement sensor, or the like. The detection portion 60 transmits the position of the outer circumferential surface of the following-material coil 3f detected by the sensor, to the control unit 100 as a detection result.

### Operations of material supply device 10

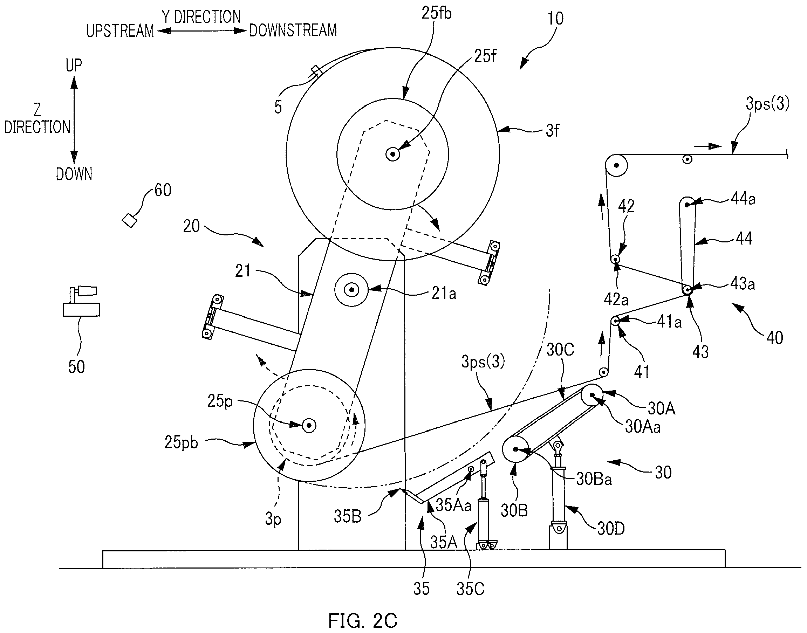

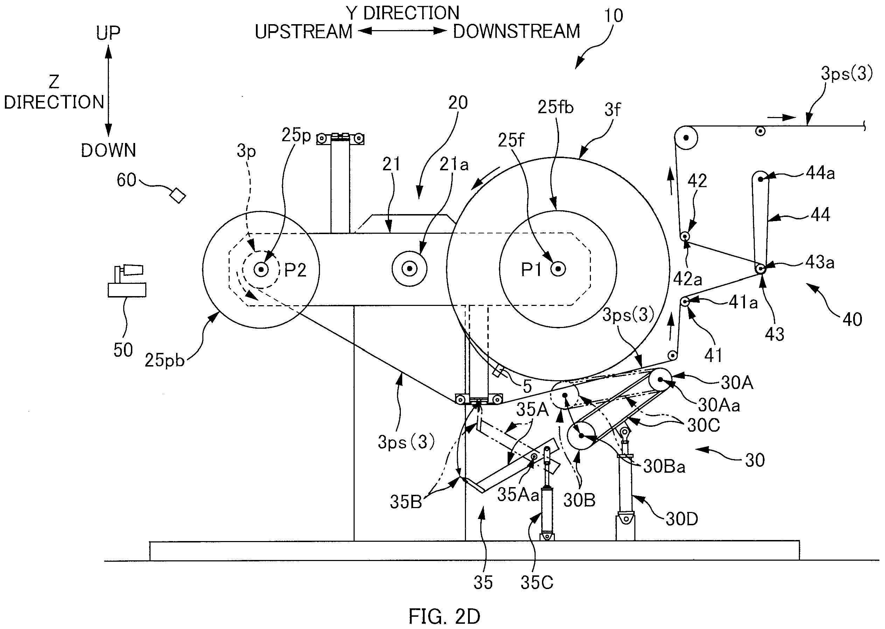

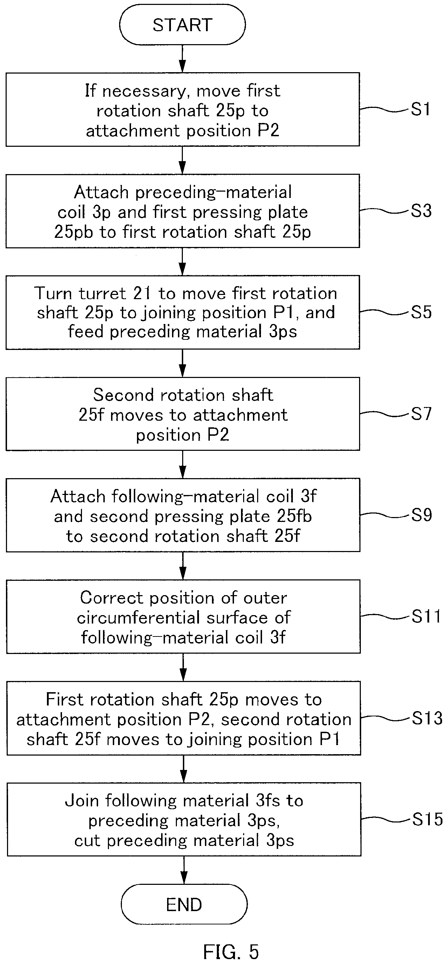

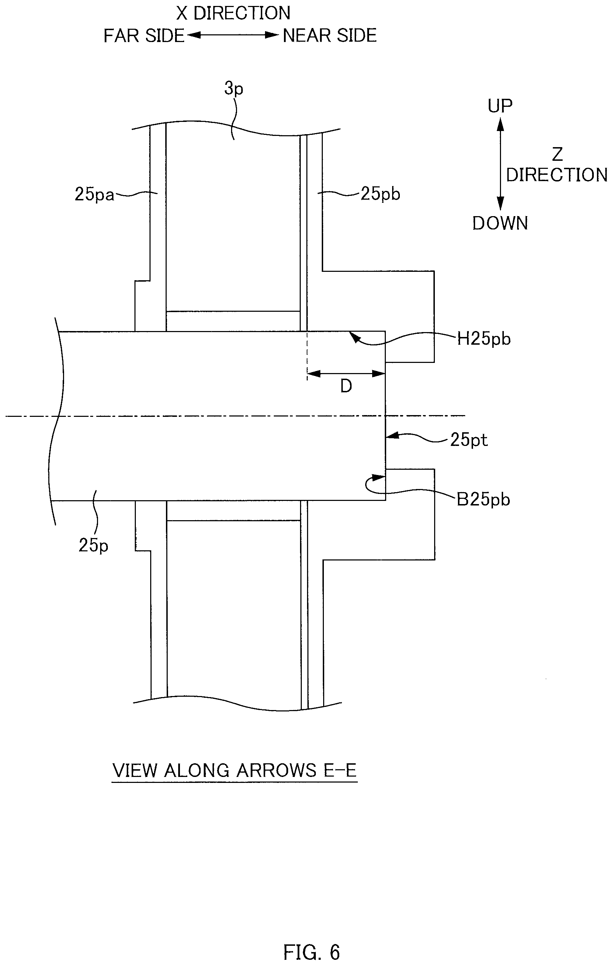

Next, operations of the material supply device 10 are described with reference to FIGS. 2A to 2D and 4 to 6. FIG. 2C is a front schematic view of the material supply device 10 in a state where the turret rotation portion 20 is turning. FIG. 2D is a front schematic view of the material supply device 10 in a state where the following material 3fs is being joined to the preceding material 3ps. FIG. 4 is a diagram showing the relationship between the control unit 100 and other devices. FIG. 5 is a flowchart showing control of operations of the material supply device 10. FIG. 6 is a cross-sectional schematic view of the first positioning portion 25pa and the first pressing plate 25pb that are attached to the first rotation shaft 25p, in a view along arrows E-E in FIG. 2A.

### Material coil attachment

First, the preceding-material coil 3p is attached to the first rotation shaft 25p, and in the present embodiment, this attachment is performed by the attachment robot 70. The attachment robot 70 of the present embodiment is a so-called articulated robot, and includes an arm that has multiple joints and an insertion portion and a grip portion provided at the leading end portion of the arm. The insertion portion is inserted into the paper tube of a material coil (the preceding-material coil 3p and the following-material coil 3f) and is for fixing the material coil from the inside of the paper tube. The grip portion is for gripping and fixing pressing plates (the first pressing plate 25pb and the second pressing plate 25fb) . The attachment robot 70 can move the fixed members by arm rotation and the like.

The attachment robot 70 is located upstream of the attachment position P2 in the Y direction, and can attach a material coil (i.e., extend the arm) to the rotation shaft that is located at the attachment position P2 (the second rotation shaft 25f in FIG. 2A) . For this reason, as shown in FIG. 2A, if the first rotation shaft 25p is located at the joining position P1, the control unit 100 turns the turret 21 so as to move the first rotation shaft 25p to the attachment position P2 (step S1).

Thereafter, the control unit 100 issues a command to the attachment robot 70 such that the attachment robot 70 retrieves a new material coil located in a material storage space (not shown) as the preceding-material coil 3p (i.e., the control unit 100 recognizes the new material coil as the preceding-material coil 3p). The preceding-material coil 3p is then moved to the attachment position P2, and the preceding-material coil 3p is attached to the first rotation shaft 25p (when the material coil is to be attached at the attachment position P2, if the correction device 50 is not located at a later-described retracted position, the control unit 100 moves the correction device 50 to the retracted position before attachment. This will be described in detail later). In other words, the first material coil (preceding-material coil 3p), which is a coil of the first material (the preceding material 3ps), is attached to the first rotation shaft 25p (corresponding to a first-material-coil attaching step).

The attachment of the preceding-material coil 3p by the attachment robot 70 is performed by matching the coil center of the preceding-material coil 3p with the rotation shaft of the first rotation shaft 25p from the near side in the X direction, and then using a pressing portion of the attachment robot 70 to press the preceding-material coil 3p from the near side to the far side in the X direction. At this time, the preceding-material coil 3p is pressed until the far-side side surface of the preceding-material coil 3p (specifically the resistant-to-deformation, paper tube of the preceding-material coil 3p) abuts against the near side of the first positioning portion 25pa (until the material coil cannot be pressed any further).

Thereafter, the attachment robot 70 grips the first pressing plate 25pb with the grip portion and attaches the first pressing plate 25pb to the first rotation shaft 25p. As shown in FIG. 6, the first pressing plate 25pb has a first-pressing-plate hole portion H25pb, which is a hole portion for insertion of the first rotation shaft 25p. A first-pressing-plate bottom face B25pb, which is the bottom face of the first-pressing-plate hole portion H25pb, has a hole portion whose diameter is smaller than the diameter of the first rotation shaft 25p. Accordingly, after the first rotation shaft 25p has entered the first-pressing-plate hole portion H25pb, the attachment robot 70 presses the first pressing plate 25pb from the near side toward the far side in the X direction until the first-pressing-plate bottom face B25pb abuts against a first rotation shaft end face 25pt, which is the near-side end face of the first rotation shaft 25p, thus attaching the first pressing plate 25pb to the first rotation shaft 25p (step S3).

In other words, in the first-material-coil attaching step, the first material coil (the preceding-material coil 3p) is attached while being sandwiched from both widthwise sides of the first material coil (the preceding-material coil 3p) between the first positioning portion 25pa and a first deformation-suppressing member (the first pressing plate 25pb) . The first positioning portion 25pa is provided at a predetermined position in the axial direction of the first rotation shaft 25p, and the first deformation-suppressing member is a member for suppressing deformation of the first material coil (the preceding-material coil 3p) in the width direction. Also, in the first-material-coil attaching step, the attachment robot 70 attaches the first material coil (the preceding-material coil 3p) to the first rotation shaft 25p so as to abut against the first positioning portion 25pa, and then after this attachment, the attachment robot 70 attaches the first deformation-suppressing member (the first pressing plate 25pb) to the first rotation shaft 25p, such that the first material coil (the preceding-material coil 3p) is sandwiched from both widthwise sides of the first material coil (the preceding-material coil 3p) between the first positioning portion 25pa and the first deformation-suppressing member (the first pressing plate 25pb) .

Here, as shown in FIG. 6, a depth D of the hole portion of the first pressing plate 25pb is a depth according to which the first pressing plate 25pb does not come into contact with the near-side side surface of the preceding-material coil 3p. In other words, the preceding-material coil 3p and the first pressing plate 25pb are attached so as to have a gap therebetween in the X direction. According to this configuration, when the preceding-material coil 3p is fed and the diameter of the preceding-material coil 3p becomes smaller than the diameter of the first positioning portion 25pa or the first pressing plate 25pb, it is possible to suppress the case where the fed preceding material 3ps excessively comes into contact with the first positioning portion 25pa or the first pressing plate 25pb.

After the attachment robot 70 has attached the preceding-material coil 3p and the first pressing plate 25pb to the first rotation shaft 25p, the turret 21 is turned by the turret rotation portion 20, thus moving the first rotation shaft 25p from the attachment position P2 to the joining position P1. The preceding material 3ps is then fed from the preceding-material coil 3p that is attached to the first rotation shaft 25p that was moved to the j oining position P1, and the material 3 is supplied to the conveying mechanism of the absorbent-article manufacturing device 1 (step S5. See the state in FIG. 2A) . Due to the turning of the turret 21, the second rotation shaft 25f moves from the joining position P1 to the attachment position P2 (step S7).

Next, the following-material coil 3f is attached to the second rotation shaft 25f. Specifically, the second material coil (following-material coil 3f), which is a coil of the second material (the following material 3fs), is attached to the second rotation shaft 25f (corresponding to a second-material-coil attaching step). This attachment is performed similarly to the above-described attachment of the preceding-material coil 3p and the first pressing plate 25pb to the first rotation shaft 25p. Specifically, the attachment robot 70 retrieves a new material coil from the material storage space as the following-material coil 3f (i.e. , the control unit 100 recognizes the new material coil as the following-material coil 3f), and attaches the following-material coil 3f so as to abut against the second positioning portion 25fa of the second rotation shaft 25f. After this attachment, the attachment robot 70 grips the second pressing plate 25fb with the grip portion and attaches the second pressing plate 25fb such that the bottom face of the hole portion of the second pressing plate 25fb abuts against the end face of the second rotation shaft 25f (step S9. See the state in FIG. 2B).

In other words, in the second-material-coil attaching step, the second material coil (the following-material coil 3f) is attached while being sandwiched from both widthwise sides of the second material coil (the following-material coil 3f) between the second positioning portion 25fa and a second deformation-suppressing member (the second pressing plate 25fb) . The second positioning portion 25fa is provided at a predetermined position in the axial direction of the second rotation shaft 25f, and the second deformation-suppressing member being a member for suppressing deformation of the second material coil (the following-material coil 3f) in the width direction. Also, in the second-material-coil attaching step, the attachment robot 70 attaches the second material coil (the following-material coil 3f) to the second rotation shaft 25p so as to abut against the second positioning portion 25fa, and then after this attachment, the attachment robot 70 attaches the second deformation-suppressing member (the second pressing plate 25fb) to the second rotation shaft 25f, such that the second material coil (the following-material coil 3f) is sandwiched from both widthwise sides of the second material coil (the following-material coil 3f) between the second positioning portion 25fa and the second deformation-suppressing member (the second pressing plate 25fb) .

### Correction of following-material coil 3f

As previously described, in the present embodiment, the following-material coil 3f is attached while being sandwiched from both widthwise sides between the second positioning portion 25fa and the second pressing plate 25fb, thus suppressing (correcting) deformation in the width direction of the following-material coil 3f. And the diameters of the second positioning portion 25fa and the second pressing plate 25fb are smaller than the diameter of the following-material coil 3f before being fed. For this reason, if the outer circumferential portion of the following-material coil 3f is misaligned by a large amount in the width direction, there are cases where such deformation cannot be sufficiently suppressed (corrected) due to the following-material coil merely being sandwiched between the second positioning portion 25fa and the second pressing plate 25fb when attached. Accordingly, after the following-material coil 3f and the second pressing plate 25fb are attached to the second rotation shaft 25f as described above, the correction device 50 performs correction (adjustment) such that the X-direction position of the outer circumferential surface of the following-material coil 3f matches the position of the preceding material 3ps (corresponding to a correcting step).

The correction device 50 performs this correction based on the detection result obtained by the detection portion 60. Specifically, the control unit 100 drives the second rotation shaft 25f to rotate such that the following-material coil 3f rotates counter-clockwise, the detection portion 60 continuously detects the X-direction position of the outer circumferential surface of the rotating following-material coil 3f, and immediately transmits the detection result to the control unit 100 (corresponding to a detecting step). Accordingly, the control unit 100 recognizes the received detection result as the X-direction position of the outer circumferential surface of the following-material coil 3f, and calculates the deviation amount between that position and the X-direction position of the preceding material 3ps with use of a calculation unit 102. This information indicating the X-direction position of the preceding material 3ps may be stored in a storage unit 101 in advance as a predetermined position sandwiched between the first positioning portion 25pa and the first pressing plate 25pb. Or the X-direction position of the preceding material 3ps may be directly detected by a sensor or the like and used as the detection result.

Thereafter, the control unit 100 moves the correction device 50 (the contact-portion support portion 52), which is located at a position radially outward of the outer circumferential surface of the following-material coil 3f (hereinafter, also called the retracted position) . This movement is performed based on the deviation amount, and the control unit 100 causes the second moving portion 54 to move in the X direction the contact-portion support portion 52, which is at the retracted position. Specifically, if the deviation amount indicates deviation to the far side (near side) in the X direction, the control unit 100 moves the contact-portion support portion 52 to a position that is on the far side (near side) in the X direction and where the outer circumferential surface of the rotating body 51a is able to come into contact with the side surface of the outer circumferential portion of the following-material coil 3f that is located at the deviated position. The control unit 100 then causes the first moving portion 53 to move the contact-portion support portion 52 downstream in the Y direction to a position at which the rotating body 51a comes into contact with the side surface of the outer circumferential portion of the following-material coil 3f (in the above description, the contact-portion support portion 52 first moves to a X-direction position at which the rotating body 51a and the following-material coil 3f can come into contact, and then moves downstream in the Y direction; but a configuration is possible in which the contact-portion support portion 52 first moves to a X-direction position at which the rotating body 51a and the following-material coil 3f are not in contact, and then moves downstream in the Y direction, and then the contact-portion support portion 52 moves in the X direction such that the rotating body 51a and the following-material coil 3f come into contact).

Once the side surface of the outer circumferential portion of the rotating following-material coil 3f comes into contact with the rotating body 51a, the control unit 100 causes the calculation unit 102 to successively calculate the deviation amount based on the information that is continuously transmitted from the detection portion 60 and indicates the X-direction position of the outer circumferential surface of the following-material coil 3f, and the control unit 100 moves the contact-portion support portion 52 (the rotating body 51a) until the deviation amount reaches zero. In other words, the position of the contact-portion support portion 52 (the rotating body 51a) is adjusted such that the deviation amount becomes zero. This position adjustment can be performed by movement of the second moving portion 54 in the X direction, or, because the rotating body 51a has a tapered shape as previously described, the position adj ustment can be performed by movement of the first moving portion 53 in the Y direction. If overcorrection occurs due to excessive adjustment (movement) (e.g., if the position is corrected from the far side, but is corrected too much and becomes misaligned on the near side), the contact-portion support portion 52 is once again moved to the retracted position, and then the contact-portion support portion 52 is moved to the opposite side of the following-material coil 3f in the X direction such that the deviation amount becomes zero.

In other words, in the correcting step, the position of the outer circumferential surface of the second rotation shaft 25f in the axial direction is corrected by the correction device 50 that comes into contact with the side surface of the outer circumferential portion and that moves the outer circumferential portion of the second material coil (the following-material coil 3f) attached to the second rotation shaft 25f from one side in the axial direction of the second rotation shaft 25f to the other side. The correction is made such that the feed position of the first material (the preceding material 3ps) in the axial direction of the first rotation shaft 25p matches the position of the outer circumferential surface of the second material coil (the following-material coil 3f) in the axial direction of the second rotation shaft 25f. Also, in the detecting step, the detection portion 60 detects the position of the outer circumferential surface of the second material coil (the following-material coil 3f) in the axial direction of the second rotation shaft 25f. In the correcting step, the correction direction and the correction amount of the correction device 50 are controlled based on information indicating the amount of deviation between the feed position of the first material (the preceding material 3ps) in the axial direction of the first rotation shaft 25p and the position of the outer circumferential surface of the second material coil (the following-material coil 3f) in the axial direction of the second rotation shaft 25f that having been detected in the detecting step.

If the deviation amount becomes zero due to correction, the control unit 100 moves the contact-portion support portion 52 to the retracted position and stops the rotation of the following-material coil 3f (step S11). The contact-portion support portion 52 is moved to the retracted position so as to not come into contact with the following-material coil 3f that is to be subsequently attached. Specifically, in the second-material-coil attaching step, the correction device 50 is moved to the retracted position located radially outward of the outer circumferential surface of the to-be-attached second material coil (the following-material coil 3f) such that the correction device 50 does not come into contact with the to-be-attached second material coil (the following-material coil 3f). In the correcting step, the correction device 50 is moved inward in the diameter direction of the second rotation shaft 25f from the retracted position such that the correction device 50 comes into contact with the side surface of the outer circumferential portion of the second material coil (the following-material coil 3f).

### Joining of preceding material 3ps and following material 3fs

While the position of the following-material coil 3f is being corrected as described above, the preceding material 3ps continues to be fed from the preceding-material coil 3p at the joining position P1. Then, after this correction, when the diameter of the preceding-material coil 3p becomes smaller than a "predetermined size for movement" due to feeding, as shown in FIG. 2C, the control unit 100 turns the turret 21 of the turret rotation portion 20 to turn so as to move the preceding-material coil 3p (the first rotation shaft 25p) from the joining position P1 to the attachment position P2, and move the following-material coil 3f (the second rotation shaft 25f) from the attachment position P2 to the joining position P1 (step S13). This "predetermined size for movement" is stored in the storage unit 101 in advance. The information that indicates the diameter of the preceding-material coil 3p that has decreased due to feeding may be calculated based on the remaining amount of the preceding material 3ps in the preceding-material coil 3p; the remaining amount being obtained by calculation by the calculation unit 102 based on integration of the amount of preceding material 3ps that has been fed based on the rotation speed of the first rotation shaft 25p the rotation time, and the like. Or, the position of the outer circumferential surface of the preceding-material coil 3p in the diameter direction may be directly detected by a provided sensor or the like.

Even after being moved to the attachment position P2, the preceding material 3ps is further fed from the preceding-material coil 3p, and when the diameter of the preceding-material coil 3p becomes smaller than a "predetermined size for joining", the control unit 100 rotates the following-material coil 3f counter-clockwise which is located at the joining position P1. This "predetermined size for joining" is stored in the storage unit 101 in advance. Also, the information indicating the diameter of the preceding-material coil 3p that has decreased due to feeding is obtained by calculation similar to the method described above, for example.

Here, as previously described, the winding end portion of the following material 3fs is located at the outer circumferential surface of the following-material coil 3f, and is fixed to the following-material coil by a joining member 5. In the following-material coil 3f rotating at the joining position P1, the winding end portion is also fixed to the following-material coil 3f by a joining member 5. Specifically, at the winding end portion of the second material (the following material 3fs) located at the outer circumferential surface of the second material coil (the following-material coil 3f), the winding end portion is fixed to the second material coil (the following-material coil 3f) by the joining member 5 so as to be prevented from separating from the second material coil (the following-material coil 3f) . The winding end portion is fixed to the following-material coil 3f such that the following material 3fs is released (the joining member 5 is peeled away) when the following material 3fs is joined to the preceding material 3ps and is fed from the following-material coil 3f.

The control unit 100 causes the following-material coil 3f located at the joining position P1 to rotate counter-clockwise, and in order to suppress variation in the tension when the following material 3fs is joined to the preceding material 3ps that is being fed, the control unit 100 adjust the rotation speed of the second rotation shaft 25f such that the rotational speed at the outer circumferential surface of the following-material coil 3f matches the feeding speed of the preceding material 3ps. When the rotational speed at the outer circumferential surface of the following-material coil 3f matches the feeding speed of the preceding material 3ps, as shown in FIG. 2D, in the connecting portion 30, the connecting-portion actuator 30D swings the swing arm upward in the Z direction to bring the belt member 30C into contact with the preceding material 3ps, and presses the preceding material 3ps against the outer circumferential surface of the rotating following-material coil 3f, thus joining the following material 3fs to the preceding material 3ps (in the present embodiment, the preceding material 3ps and the following material 3fs are joined using a joining member provided on the outer side of the winding end portion of the following-material coil 3f). In other words, the first material (the preceding material 3ps) that is being fed from the first material coil (the preceding-material coil 3p) due to driven rotation of the first rotation shaft 25p is pressed against the outer circumferential surface of the second material coil (the following-material coil 3f) that is rotating due to driven rotation of the second rotation shaft 25f, thus joining the second material (the following material 3fs) to the first material (the preceding material 3ps) (corresponding to a joining step).

Also, in the cutting portion 35, after the following material 3fs is joined to the preceding material 3ps, the cutting-portion actuator 35C swings the arm member 35A upward in the Z direction, and the preceding material 3ps is cut by the cutter blade 35B (step S15). Specifically, without stopping the feeding of the preceding material 3ps that has been supplied from the material supply device 10 to the conveying mechanism, the preceding material 3ps is changed to the following material 3fs that is fed from the following-material coil 3f. In other words, without stopping the feeding of the first material (the preceding material 3ps) that has been continuously fed from the preceding first material coil (the preceding-material coil 3p), the second material (the following material 3fs) supplied from the following second material coil (the following-material coil 3f) is joined to the first material (the preceding material 3ps), and the second material (the following material 3fs) is then fed.

When the material 3 fed to the conveying mechanism has been changed from the preceding material 3ps to the following material 3fs, the control unit 100 recognizes the following material 3fs (the following-material coil 3f) being fed on the second rotation shaft 25f as the preceding material 3ps (the preceding-material coil 3p) and overwrites the information in the storage unit 101. Accordingly, if another following-material coil 3f needs to be attached (if the following material 3fs needs to be joined to the preceding material 3ps again), a new material coil is attached to the first rotation shaft 25p located at the attachment position P2 as the following-material coil 3f. The above-described correction, joining, and the like performed with respect to the following-material coil 3f attached to the second rotation shaft 25f is similarly performed on the following-material coil 3f that has been attached to the first rotation shaft 25p. In this way, new material coils are alternatingly attached, as the following-material coil 3f, to the first rotation shaft 25p or the second rotation shaft 25f located at the attachment position P2, and correction, joining, and the like are performed on the attached following-material coil 3f, and therefore it is possible to join the following material 3fs to the preceding material 3ps without stopping the feeding of the preceding material 3ps, and continue to feed the following material 3fs to the conveying mechanism.

### Effectiveness of present embodiment

As described above, the method for manufacturing an absorbent article according to the present embodiment is a method for manufacturing an absorbent article in which, without stopping feeding of the preceding material 3ps that has been continuously fed from the preceding-material coil 3p, the following material 3fs supplied from the following-material coil 3f is joined to the preceding material 3ps, then feeding the following material 3fs, the method for manufacturing an absorbent article including: a first-material-coil attaching step of attaching the preceding-material coil 3p to the first rotation shaft 25p, the preceding material 3ps being wound around the preceding-material coil 3p; a second-material-coil attaching step of attaching the following-material coil 3f to the second rotation shaft 25f, the following material 3fs being wound around the following-material coil 3f; and a joining step of joining the following material 3fs to the preceding material 3ps by pressing the preceding material 3ps against the outer circumferential surface of the following-material coil 3f, the preceding material 3ps being fed from the preceding-material coil 3p due to driven rotation of the first rotation shaft 25p, the following-material coil 3f rotating due to driven rotation of the second rotation shaft 25f. The size of the preceding-material coil 3p in the diameter direction is larger than the size of the preceding-material coil 3p in the width direction. The size of the following-material coil 3f in the diameter direction is larger than the size of the following-material coil 3f in the width direction. In the first-material-coil attaching step, the preceding-material coil 3p is attached while being sandwiched from two widthwise sides of the preceding-material coil 3p between the first positioning portion 25pa and the first pressing plate 25pb, the first positioning portion 25pa being provided at a predetermined position in the axial direction of the first rotation shaft 25p, the first pressing plate 25pb being a member for suppressing widthwise deformation of the preceding-material coil 3p. In the second-material-coil attaching step, the following-material coil 3f is attached while being sandwiched from two widthwise sides of the following-material coil 3f between the second positioning portion 25fa and the second pressing plate 25fb, the second positioning portion 25fa being provided at a predetermined position in the axial direction of the second rotation shaft 25f, the second pressing plate 25fb being a member for suppressing widthwise deformation of the following-material coil 3f. Accordingly, it is possible to suppress misalignment when joining the preceding material 3ps and the following material 3fs.

When the following material 3fs supplied from the following-material coil 3f is joined to the preceding material 3ps that is continuously fed from the preceding-material coil 3p without stopping the feeding of the preceding material 3ps, and the following material 3fs is then fed, conventionally, no consideration whatsoever has been given to misalignment between the preceding material 3ps and the following material 3fs in the width direction. For this reason, in the case where the material coil of the material 3 is constituted solely by a highly flexible material and has a diameter that is much larger than the size in the width direction as in the present embodiment, the problem of misalignment of the following material 3fs joined to the preceding material 3ps in the width direction has occurred frequently.

To address this, in the method for manufacturing an absorbent article according to the present embodiment, the preceding-material coil 3p (the following-material coil 3f) is attached while being sandwiched from both sides of the preceding-material coil 3p (the following-material coil 3f) in the width direction, by the first positioning portion 25pa (second positioning portion 25fa) provided at a predetermined position in the axial direction of the first rotation shaft 25p (second rotation shaft 25f) and the first pressing plate 25pb (the second pressing plate 25fb) for suppressing deformation of the preceding-material coil 3p (the following-material coil 3f) in the width direction. In other words, even if the preceding-material coil 3p (the following-material coil 3f) attached to the first rotation shaft 25p (the second rotation shaft 25f) attempts to deform in the width direction, the deformation of the preceding-material coil 3p (the following-material coil 3f) in the width direction is suppressed due to contact with the first positioning portion 25pa (the second positioning portion 25fa) or the first pressing plate 25pb (the second pressing plate 25fb). Accordingly, in the present embodiment, it is possible to suppress misalignment when joining the preceding material 3ps and the following material 3fs.

Also, in the present embodiment, the first deformation-suppressing member (the second deformation-suppressing member) is the first pressing plate 25pb (the second pressing plate 25fb) that has a predetermined length in the diameter direction of the first rotation shaft 25p (the second rotation shaft 25f), and the predetermined length of the first pressing plate 25pb (the second pressing plate 25fb) is smaller than the radius of the preceding-material coil 3p (the following-material coil 3f) before being fed as the preceding material 3ps (the following material 3fs). Also, the first positioning portion 25pa (the second positioning portion 25fa) is plate-shaped and has a predetermined length in the diameter direction of the first rotation shaft 25p (the second rotation shaft 25f), and the predetermined length of the first positioning portion 25pa (the second positioning portion 25fa) is smaller than the radius of the preceding-material coil 3p (the following-material coil 3f) before being fed as the preceding material 3ps (the following material 3fs).

In other words, in the present embodiment, in order for the preceding material 3ps to be reliably pressed against and joined to the following-material coil 3f, and therefore the width of the belt member 30C is larger than the width of the preceding material 3ps and the following-material coil 3f. For this reason, the radii of the first pressing plate 25pb (the second pressing plate 25fb) and the first positioning portion 25pa (the second positioning portion 25fa) are set smaller than the radius of the preceding-material coil 3p (the following-material coil 3f) before being fed as the preceding material 3ps (the following material 3fs), and therefore when the belt member 30C swings upward in the Z direction and presses the preceding material 3ps against the following-material coil 3f so as to join the preceding material 3ps and the following material 3fs, it is possible to suppress the case where the belt member 30C comes into contact with the first pressing plate 25pb (the second pressing plate 25fb) and the first positioning portion 25pa (the second positioning portion 25fa).

Also, in the present embodiment, the first deformation-suppressing member (the second deformation-suppressing member) is the first pressing plate 25pb (the second pressing plate 25fb) that has a predetermined length in the diameter direction of the first rotation shaft 25p (the second rotation shaft 25f), and the predetermined length of the first pressing plate 25pb (the second pressing plate 25fb) is larger than 1/3 of the radius of the preceding-material coil 3p (the following-material coil 3f) before being fed as the preceding material 3ps (the following material 3fs) .

In other words, depending on the type of material used as the preceding material 3ps (the following material 3fs), the first pressing plate 25pb (the second pressing plate 25fb) has a different radius in order to be effective in suppressing deformation of the preceding-material coil 3p (the following-material coil 3f) in the width direction, and if the radius of the first pressing plate 25pb (the second pressing plate 25fb) is smaller than 1/3 of the radius of the preceding-material coil 3p (the following-material coil 3f) before being fed as the preceding material 3ps (the following material 3fs), it is not possible to expect a sufficient effect of suppressing deformation of the preceding material 3ps (the following material 3fs) in the width direction with use of the first pressing plate 25pb (the second pressing plate 25fb) regardless of the type of material used as the preceding material 3ps (the following material 3fs) .

Also, when the position of the outer circumferential surface of the following-material coil 3f is corrected by the correction device 50, if the radius of the first pressing plate 25pb (the second pressing plate 25fb) is smaller than 1/3 of the radius of the preceding-material coil 3p (the following-material coil 3f) before being fed, there are cases where the outer circumferential portion of the following-material coil 3f swerves in the width direction due to driven rotation of the first rotation shaft 25p (the second rotation shaft 25f), and it is difficult for the correction device 50 to perform correction in such cases. For this reason, by setting the radius of the first pressing plate 25pb (the second pressing plate 25fb) larger than 1/3 of the radius of the preceding-material coil 3p (the following-material coil 3f) before being fed, it is possible to further suppress misalignment of the material coils in the width direction.

Also, in the present embodiment, the method for manufacturing an absorbent article further includes a correcting step of correcting the position of the outer circumferential surface of the following-material coil 3f in the axial direction of the second rotation shaft 25f. The correcting being performed such that the feeding position of the preceding material 3ps in the axial direction of the first rotation shaft 25p matches the position of the outer circumferential surface of the following-material coil 3f in the axial direction of the second rotation shaft 25f. The correcting being performed by the correction device 50 configured to perform correction by coming into contact with the side surface of the outer circumferential portion of the following-material coil 3f attached to the second rotation shaft 25f and moving the outer circumferential portion from one side toward the other side in the axial direction of the second rotation shaft 25f. In other words, the position of the outer circumferential surface of the following-material coil 3f in the X direction can be directly corrected to match the feeding position of the preceding material 3ps. Accordingly, it is possible to further suppress misalignment when joining the preceding material 3ps and the following material 3fs.

Also, in the present embodiment, at least one of the correction device 50 is provided for the second rotation shaft 25f, and the correction device 50 includes the first moving portion 53 configured to move along the diameter direction of the second rotation shaft 25f. In the second-material-coil attaching step, the correction device 50 is retracted to the retracted position so as not to come into contact with the following-material coil 3f that is to be attached, the retracted position being a position that is outward in the diameter direction with respect to the outer circumferential surface of the following-material coil 3f that is to be attached. In the correcting step, the correction device 50 is moved inward in the diameter direction from the retracted position of the second rotation shaft 25f so as to come into contact with the side surface of the outer circumferential portion of the following-material coil 3f. In other words, the correction device 50 can move in the diameter direction of the second rotation shaft 25f, and thus retracts outward in the diameter direction so as to not come into contact with the following-material coil 3f when the following-material coil 3f is to be attached, and moves inward in the diameter direction so as to come into contact with the outer circumferential portion of the following-material coil 3f when the correcting the position of the outer circumferential surface of the following-material coil 3f. For this reason, it is possible to suppress the case where the following-material coil 3f comes into contact with the correction device 50 during attachment of the following-material coil 3f.

Also, in the present embodiment, the contact portion 51 of the correction device 50 that comes into contact with the following-material coil 3f is constituted by the rotating body 51a that can rotate about a rotation axis extending along the diameter direction of the second rotation shaft 25f. In other words, compared to the case where the contact portion 51 cannot rotate, it is possible to suppress frictional force between the contact portion 51 and the following-material coil 3f.

Also, in the present embodiment, the rotating body 51a has a tapered shape according to which the diameter of the rotating body 51a increases from the inward side toward the outward side in the diameter direction of the second rotation shaft 25f. In other words, the correction amount is different between the inward side and the outward side in the diameter direction of the rotating body 51a, and the correction can be made a larger amount outside than inside in the outer circumferential direction of the following-material coil 3f. In other words, that correction can be made a larger amount outside than inside in the outer circumferential direction of the following-material coil 3f makes it possible to effectively perform larger correction on the outer circumferential portion of the following-material coil 3f on the side nearer to the outer circumferential surface (the outward side in the diameter direction).

Also, in the present embodiment, the method for manufacturing an absorbent article further includes a detecting step of detecting the position of the outer circumferential surface of the following-material coil 3f in the axial direction of the second rotation shaft 25f with use of the detection portion 60. In the correcting step, the correction direction and the correction amount of the correction device 50 are controlled based on information indicating the amount of deviation between the feeding position of the preceding material 3ps in the axial direction of the first rotation shaft 25f and the position of the outer circumferential surface of the following-material coil 3f in the axial direction of the second rotation shaft 25f, the position having been detected in the detecting step. In other words, the deviation amount can be calculated by the calculation unit 102 based on the continuous detection results from the detection portion 60 and the information indicating the feeding position of the preceding material 3ps, and the control unit 100 can adjust (control) the correction direction and the correction amount of the correction device 50 using the deviation amount. In other words, compared to the case where such adjustment is performed manually, adjustment can be performed more quickly and accurately, and it is possible to further suppress misalignment when the preceding material 3ps and the following material 3fs are joined.

Also, in the present embodiment, the winding end portion of the following material 3fs is located at the outer circumferential surface of the following-material coil 3f, and the winding end portion is fixed to the following-material coil 3f using the joining member 5 so as to be prevented from separating from the following-material coil 3f. In other words, the winding end portion of the following material 3fs can remain fixed to the following-material coil 3f until the following material 3fs is joined to the preceding material 3ps and then the following material 3fs begins to be fed, thus making it possible to suppress misalignment of the winding end portion in the width direction.

Also, in the present embodiment, in the first-material-coil attaching step (the second-material-coil attaching step), the attachment robot 70 attaches the preceding-material coil 3p (the following-material coil 3f) to the first rotation shaft 25p (the second rotation shaft 25f) so as to abut against the first positioning portion 25pa (the second positioning portion 25fa), and then after the attachment, the attachment robot 70 attaches the first pressing plate 25pb (the second pressing plate 25fb) to the first rotation shaft 25p (the second rotation shaft 25f) such that the preceding-material coil 3p (the following-material coil 3f) is sandwiched from two widthwise sides of the preceding-material coil 3p (the following-material coil 3f) between the first positioning portion 25pa (the second positioning portion 25fa) and the first pressing plate 25pb (the second pressing plate 25fb). In other words, compared to the case where such operations are performed manually, the material coil can be more quickly attached while being sandwiched between the positioning portion and the pressing plate.

### Other embodiments

Although the embodiment of the present disclosure has been described hereinabove, the above embodiments of the present disclosure is simply to facilitate understanding of the present disclosure and are not in any way to be construed as limiting the present disclosure. The present disclosure may variously be changed or altered without departing from its gist and encompass equivalents thereof.

Also, in the above embodiment, the following-material coil 3f rotates in the state where the correction device 50 is in contact with the outer circumferential portion of the following-material coil 3f, thereby correcting the entire circumference of the outer circumferential portion of the following-material coil 3f, but the present invention is not limited to this. For example, the correction device 50 may move along the circumferential direction of the following-material coil 3f, centered on the second rotation shaft 25f. In other words, a configuration is possible in which in the correcting step, the correction device 50 performs correction over the entire circumference of the outer circumferential portion of the second material coil (the following-material coil 3f) using at least either one of the following methods: a method in which the second material coil (the following-material coil 3f) is rotated around the second rotation shaft 25f in a state where the correction device 50 is fixed at a predetermined position in the circumferential direction of the second material coil (the following-material coil 3f) ; and a method in which a rotation moving portion is provided for moving the correction device 50 along the circumferential direction of the second material coil (the following-material coil 3f) centered about the second rotation shaft 25f, and the correction device 50 is moved along the circumferential direction of the second material coil (the following-material coil 3f) by the rotation moving portion in a state where rotation of the second material coil (the following-material coil 3f) is temporarily stopped,.

Also, in the above embodiment, the following-material coil 3f rotates due to driven rotation of the second rotation shaft 25f in the state where the correction device 50 is in contact with the outer circumferential portion of the following-material coil 3f, thereby correcting the entire circumference of the outer circumferential portion of the following-material coil 3f, but the present invention is not limited to this . For example, a configuration is possible in which the rotating-body support portion 51b of the correction device 50 is provided with a motor or the like as a motive power source for driving rotation, and the following-material coil 3f is rotated by driving the rotating body 51a to rotate. Specifically, the correction device 50 may correct the entire circumference of the outer circumferential portion of the following-material coil 3f by rotating the second material coil (the following-material coil 3f) around the second rotation shaft 25f using at least one of the following two methods: a method of rotating the second material coil (the following-material coil 3f) by driven rotation of the second rotation shaft 25f to which the second material coil (the following-material coil 3f) is attached, and a method in which the contact portion (the contact portion 51) of the correction device 50 that comes into contact with the second material coil (the following-material coil 3f) is constituted by the rotating body 51a that can rotate about a rotation axis that extends in the diameter direction of the second rotation shaft 25f, and the second material coil (the following-material coil 3f) rotates due to frictional force between the rotating body 51a and the second material coil (the following-material coil 3f) when the rotating body 51a is driven to rotate.

The method by which the correction device 50 corrects the entire circumference of the outer circumferential portion of the following-material coil 3f is a method in which the correction device 50 moves along the circumferential direction of the following-material coil 3f centered about the second rotation shaft 25f, or a method in which the following-material coil 3f rotates due to driven rotation of the rotating body 51a, and, as previously described, because the diameter the second pressing plate 26pb is small, there are cases where the outer circumferential portion of the following-material coil 3f swerves in the width direction when the following-material coil 3f rotates due to driven rotation of the second rotation shaft 25f, and it is difficult for the correction device 50 to perform correction in such a case, but according to the above embodiment, the correction device 50 can perform correction because the following-material coil 3f does not rotate due to driven rotation of the second rotation shaft 25f (because the outer circumferential portion does not swerve in the width direction while rotating). In other words, the correction device 50 can perform correction over the entire circumference of the outer circumferential portion of the following-material coil 3f.

Also, in the above embodiment, the first positioning portion 25pa, the second positioning portion 25fa, the first pressing plate 25pb, and the second pressing plate 25fb are approximately circular and have a predetermined length in the diameter direction that is approximately the same over the entire circumference, but the present invention is not limited to this. For example, these members may be elliptical or rectangular, or may be approximately circular with a protruding or receding portion in part of the circular shape.

Also, in the above embodiment, the predetermined lengths of the first positioning portion 25pa, the second positioning portion 25fa, the first pressing plate 25pb, and the second pressing plate 25fb are set smaller than the radius of the material coils before being fed in order to prevent contact with the belt member 30C when joining is performed, but the present invention is not limited to this. For example, a configuration is possible in which the predetermined lengths are set larger than the radius, and the width of the belt member 30C is set smaller than the widths of the material coils. However, the above embodiment is desirable in view of being able to reliably join the following material 3fs to the preceding material 3ps.

Also, although the material coils are constituted by highly flexible nonwoven fabric and have a larger size in the diameter direction than in the width direction in the above embodiment, the present invention is not limited to this. For example, the present invention is applicable to any material coil that can easily deform in the width direction, even if the material coil is constituted by a film or the like in addition to nonwoven fabric, or does not include nonwoven fabric, or has a larger size in the width direction than in the diameter direction.

Also, although one correction device 50 is provided for the second rotation shaft 25f in the above embodiment, the present invention is not limited to this. For example, one correction device 50 may be provided on each side in the width direction of the following-material coil 3f.

Also, although the correction device 50 and the detection portion 60 are provided at the attachment position P2 in the above embodiment, the present invention is not limited to this. For example, a configuration is possible in which the correction device 50 and the detection portion 60 are provided at the joining position P1, and the correction device 50 corrects the position of the outer circumferential surface of the following-material coil 3f after the diameter of the preceding-material coil 3p has fallen below the "predetermined size for movement" and the following-material coil 3f has moved to the joining position P1 due to the turning of the turret 21.

Although the connecting portion 30 and the cutting portion 35 are fixed at predetermined positions in the Z direction in the above embodiment, the present invention is not limited to this. For example, if the predetermined length of the first pressing plate 25pb in the diameter direction is larger than in the above embodiment, when the turret 21 turns as shown in FIG. 2C, there is a risk that the first pressing plate 25pb will come into contact with the connecting portion 30 or the cutting portion 35. A configuration is possible in which a vertical moving portion is provided for moving the connecting portion 30 and the cutting portion 35 back and forth in the Z direction, and the connecting portion 30 and the cutting portion 35 are moved upward in the Z direction so as to approach the preceding material 3ps only when joining is to be performed (on in the state shown in FIG. 2D).

Also, the position of the outer circumferential surface of the following-material coil 3f in the X direction is detected by the detection portion 60 in the above embodiment, and as one specific example, the detection portion 60 can be the laser displacement sensor IL-S100 (Keyence Corporation). However, the present invention is not limited to this.

Also, although the preceding-material coil 3p is attached to the first rotation shaft 25p at the attachment position P2 in the above embodiment, the present invention is not limited to this. For example, a configuration is possible in which the arm of the attachment robot 70 can reach the joining position P1, and the preceding-material coil 3p is attached to the first rotation shaft 25p at the joining position P1, or the preceding-material coil 3p is manually attached at the joining position P1.

Also, although the following material 3fs is joined to the preceding material 3ps by a joining member provided on the outer circumferential surface of the following-material coil in the above embodiment, the present invention is not limited to this. For example, this joining may be performed by heat sealing or the like.

### [Reference Signs List]

1 absorbent-article manufacturing device, 2 absorbent body, 3 material,
3p preceding-material coil
3ps preceding material, 3f following-material coil, 3fs following material, 4 processing unit
5 joining member, 10 material supply device, 20 turret rotation portion, 21 turret
21a turret shaft, 25p first rotation shaft, 25pt first rotation shaft end face
25pa first positioning portion, 25pb first pressing plate, H25pb first-pressing-plate hole portion
B25pb first-pressing-plate bottom face, 25f second rotation shaft, 25fa second positioning portion
25pb second pressing plate, 30 connecting portion, 35 cutting portion,
40 accumulating device
50 correction device, 51 contact portion, 51a rotating body, 51b rotating-body support portion
52 contact-portion support portion, 53 first moving portion, 54 second moving portion, 60 detection portion
70 attachment robot, 100 control unit, 101 storage unit, 102 calculation unit
P1 joining position, P2 attachment position

## Claims

1. A method for manufacturing an absorbent article in which, without stopping feeding of a first material (3ps) that has been continuously fed from a preceding first material coil (3p), a second material (3fs) supplied from a following second material coil (3f) is joined to the first material (3ps), then feeding the second material (3fs),
the method for manufacturing the absorbent article comprising:
a first-material-coil attaching step of attaching the first material coil (3p) to a first rotation shaft, the first material being wound around the first material coil (3p);
a second-material-coil attaching step of attaching a second material coil (3f) to a second rotation shaft, the second material (3fs) being wound around the second material coil (3f); and
a joining step of joining the second material (3fs) to the first material (3ps) by pressing the first material (3ps) against an outer circumferential surface of the second material coil (3f),
the first material (3ps) being fed from the first material coil (3p) due to driven rotation of the first rotation shaft,
the second material coil (3f) rotating due to driven rotation of the second rotation shaft,
a size of the first material coil (3p) in a diameter direction being larger than a size of the first material coil (3p) in a width direction,
a size of the second material coil (3f) in a diameter direction being larger than a size of the second material coil (3f) in a width direction,
in the first-material-coil attaching step,
the first material coil (3p) being attached while being sandwiched from two widthwise sides of the first material coil (3p) between a first positioning portion (25pa) and a first deformation-suppressing member,
the first positioning portion (25pa) being provided at a predetermined position in an axial direction of the first rotation shaft,
the first deformation-suppressing member being a member for suppressing widthwise deformation of the first material coil (3p),
in the second-material-coil attaching step,
the second material coil (3f) being attached while being sandwiched from two widthwise sides of the second material coil (3f) between a second positioning portion (25fa) and a second deformation-suppressing member,
the second positioning portion (25fa) being provided at a predetermined position in an axial direction of the second rotation shaft,
the second deformation-suppressing member being a member for suppressing widthwise deformation of the second material coil (3f),
wherein
the first deformation-suppressing member is a first pressing plate (25pb) that has a predetermined length in a diameter direction of the first rotation shaft, and
the predetermined length of the first pressing plate (25pb) is larger than 113 of a radius of the first material coil (3p) before being fed as the first material (3ps), and
wherein
the second deformation-suppressing member is a second pressing plate (25fb) that has a predetermined length in a diameter direction of the second rotation shaft, and
the predetermined length of the second pressing plate (25fb) is larger than 113 of a radius of the second material coil (3f) before being fed as the second material (3fs).

2. The method for manufacturing an absorbent article according to claim 1, wherein
the first deformation-suppressing member is a first pressing plate (25pb) that has a predetermined length in a diameter direction of the first rotation shaft, and
the predetermined length of the first pressing plate (25pb) is smaller than a radius of the first material coil (3p) before being fed as the first material (3ps).
first material coil (3p)

3. The method for manufacturing an absorbent article according to any one of claims 1 to 2, wherein
the second deformation-suppressing member is a second pressing plate (25fb) that has a predetermined length in a diameter direction of the second rotation shaft, and
the predetermined length of the second pressing plate (25fb) is smaller than a radius of the second material coil (3f) before being fed as the second material (3fs). second material coil (3f)

4. The method for manufacturing an absorbent article according to any one of claims 1 to 3, wherein
the first positioning portion (25pa) is plate-shaped and has a predetermined length in a diameter direction of the first rotation shaft, and
the predetermined length of the first positioning portion (25pa) is smaller than a radius of the first material coil (3p) before being fed as the first material (3ps).

5. The method for manufacturing an absorbent article according to any one of claims 1 to 4, wherein
the second positioning portion (25fa) is plate-shaped and has a predetermined length in a diameter direction of the second rotation shaft, and
the predetermined length of the second positioning portion (25fa) is smaller than a radius of the second material coil (3f) before being fed as the second material (3fs).

6. The method for manufacturing an absorbent article according to any one of claims 1 to 5, wherein
the method further comprises a correcting step of correcting a position of the outer circumferential surface of the second material coil (3f) in an axial direction of the second rotation shaft,
the correcting being performed such that a feeding position of the first material (3ps) in an axial direction of the first rotation shaft matches the position of the outer circumferential surface of the second material coil (3f) in the axial direction of the second rotation shaft,
the correcting being performed by a correction device configured to perform correction by coming into contact with a side surface of an outer circumferential portion of the second material coil (3f) attached to the second rotation shaft and moving the outer circumferential portion from one side toward another side in the axial direction of the second rotation shaft.

7. The method for manufacturing an absorbent article according to claim 6, wherein
at least one of the correction device is provided for the second rotation shaft,
the correction device includes a moving portion configured to move along a diameter direction of the second rotation shaft,
in the second-material-coil attaching step,
the correction device is retracted to a retracted position so as not to come into contact with the second material coil (3f) that is to be attached,
the retracted position being a position that is outward in the diameter direction with respect to the outer circumferential surface of the second material coil (3f) that is to be attached, and
in the correcting step,
the correction device is moved inward in the diameter direction of the second rotation shaft from the retracted position so as to come into contact with the side surface of the outer circumferential portion of the second material coil (3f).

8. The method for manufacturing an absorbent article according to claim 6 or 7, wherein
a contact portion of the correction device that comes into contact with the second material coil (3f) is constituted by a rotating body that can rotate about a rotation axis extending along a diameter direction of the second rotation shaft.

9. The method for manufacturing an absorbent article according to claim 8, wherein
the rotating body has a tapered shape according to which a diameter of the rotating body increases from an inward side toward an outward side in the diameter direction of the second rotation shaft.

10. The method for manufacturing an absorbent article according to any one of claims 7 to 9, wherein
in the correcting step,
the correction device performs correction over an entire circumference of the outer circumferential portion of the second material coil (3f) using at least either one of
a method in which the second material coil (3f) is rotated around the second rotation shaft in a state where the correction device is fixed at a predetermined position in a circumferential direction of the second material coil (3f), and
a method in which a rotation moving portion is provided for movement of the correction device along the circumferential direction of the second material coil (3f) centered about the second rotation shaft, and in which the correction device is moved along the circumferential direction of the second material coil (3f) by the rotation moving portion in a state where rotation of the second material coil (3f) is temporarily stopped.

11. The method for manufacturing an absorbent article according to any one of claims 6 to 10, wherein
the method further comprises a detecting step of detecting the position of the outer circumferential surface of the second material coil (3f) in the axial direction of the second rotation shaft with use of a detection portion, and
in the correcting step,
a correction direction and a correction amount of the correction device are controlled based on information indicating an amount of deviation between
the feeding position of the first material (3ps) in the axial direction of the first rotation shaft and
the position of the outer circumferential surface of the second material coil (3f) in the axial direction of the second rotation shaft, the position having been detected in the detecting step.

12. The method for manufacturing an absorbent article according to any one of claims 1 to 11, wherein
a winding end portion of the second material (3fs) is located at the outer circumferential surface of the second material coil (3f), and
the winding end portion is fixed to the second material coil (3f) using a joining member so at to be prevented from separating from the second material coil (3f).

13. The method for manufacturing an absorbent article according to any one of claims 1 to 12, wherein
in the first-material-coil attaching step,
an attachment robot attaches the first material coil (3p) to the first rotation shaft so as to abut against the first positioning portion (25pa), and
then after the attachment, the attachment robot attaches the first deformation-suppressing member to the first rotation shaft such that the first material coil (3p) is sandwiched from two widthwise sides of the first material coil (3p) by the first positioning portion (25pa) and the first deformation-suppressing member, and
in the second-material-coil attaching step,
the attachment robot attaches the second material coil (3f) to the second rotation shaft so as to abut against the second positioning portion (25fa), and
then after the attachment, the attachment robot attaches the second deformation-suppressing member to the second rotation shaft such that the second material coil (3f) is sandwiched from two widthwise sides of the second material coil (3f) by the second positioning portion (25fa) and the second deformation-suppressing member.

14. A device for manufacturing an absorbent article, comprising:
a first material coil (3p) around which a first material (3ps) is wound,
a size of the first material coil (3p) in a diameter direction being larger than a size of the first material coil (3p) in a width direction;
a second material coil (3f) around which a second material (3fs) is wound,
a size of the second material coil (3f) in a diameter direction being larger than a size of the second material coil (3f) in a width direction,
without stopping feeding of the first material (3ps) that has been continuously fed from the preceding first material coil (3p), the second material (3fs) supplied from the following second material coil (3f) being joined to the first material (3ps) and then being fed;
a first rotation shaft to which a first material coil (3p) is to be attached;
a connecting portion configured to join the second material (3fs) to the first material (3ps) by, through driven rotation of the first rotation shaft, pressing the first material (3ps) fed from the first material coil (3p) against an outer circumferential surface of the second material coil (3f) that is rotating due to driven rotation of the second rotation shaft,
a first positioning portion (25pa) that is provided at a predetermined position in an axial direction of the first rotation shaft;
a first deformation-suppressing member configured to suppress widthwise deformation of the first material coil (3p),
the first positioning portion (25pa) and the first deformation-suppressing member being provided in order to sandwich the first material coil (3p) from two widthwise sides of the first material coil (3p) during attachment;
a second positioning portion (25fa) that is provided at a predetermined position in an axial direction of the second rotation shaft; and
a second deformation-suppressing member configured to suppress widthwise deformation of the second material coil (3f),
the second positioning portion (25fa) and the second deformation-suppressing member being provided in order to sandwich the second material coil (3f) from two widthwise sides of the second material coil (3f) during attachment,
wherein
the first deformation-suppressing member is a first pressing plate (25pb) that has a predetermined length in a diameter direction of the first rotation shaft, and
the predetermined length of the first pressing plate (25pb) is larger than 1/3 of a radius of the first material coil (3p) before being fed as the first material (3ps), and
wherein
the second deformation-suppressing member is a second pressing plate (25fb) that has a predetermined length in a diameter direction of the second rotation shaft, and
the predetermined length of the second pressing plate (25fb) is larger than 113 of a radius of the second material coil (3f) before being fed as the second material (3fs).

## Patentansprüche

1. Verfahren zum Herstellen eines saugfähigen Artikels, bei dem ohne Unterbrechung der Zufuhr eines ersten Materials (3ps), das kontinuierlich von einer vorangehenden ersten Materialspule (3p) zugeführt worden ist, ein zweites Material (3fs), das von einer folgenden zweiten Materialspule (3f) geliefert wird, mit dem ersten Material (3ps) verbunden wird, dann das zweite Material (3fs) zugeführt wird,
wobei das Verfahren zum Herstellen des saugfähigen Artikels Folgendes umfasst:
einen Schritt des Anbringens der ersten Materialspule zum Anbringen der ersten Materialspule (3p) an einer ersten Drehwelle, wobei das erste Material um die erste Materialspule (3p) gewickelt wird;
einen Schritt des Anbringens der zweiten Materialspule zum Anbringen einer zweiten Materialspule (3f) an einer zweiten Drehwelle, wobei das zweite Material (3fs) um die zweite Materialspule (3f) gewickelt wird; und
einen Verbindungsschritt zum Verbinden des zweiten Materials (3fs) mit dem ersten Material (3ps), indem das erste Material (3ps) gegen eine Außenumfangsfläche der zweiten Materialspule (3f) gedrückt wird,
wobei das erste Material (3ps) von der ersten Materialspule (3p) aufgrund von angetriebener Drehung der ersten Drehwelle zugeführt wird,
wobei sich die zweite Materialspule (3f) aufgrund von angetriebener Drehung der zweiten Drehwelle dreht,
wobei eine Größe der ersten Materialspule (3p) in einer Durchmesserrichtung größer als eine Größe der ersten Materialspule (3p) in einer Breitenrichtung ist,
wobei eine Größe der zweiten Materialspule (3f) in einer Durchmesserrichtung größer als eine Größe der zweiten Materialspule (3f) in einer Breitenrichtung ist,
wobei in dem Schritt des Anbringens der ersten Materialspule
die erste Materialspule (3p) angebracht wird, während sie von zwei Breitenseiten der ersten Materialspule (3p) zwischen einem ersten Positionierungsabschnitt (25pa) und
einem ersten Verformungsunterdrückungselement eingefügt ist,
der erste Positionierungsabschnitt (25pa) an einer vorbestimmten Position in einer axialen Richtung der ersten Drehwelle bereitgestellt ist,
das erste Verformungsunterdrückungselement ein Element zum Unterdrücken von Breitenverformung der ersten Materialspule (3p) ist,
wobei in dem Schritt des Anbringens der zweiten Materialspule die zweite Materialspule (3f) angebracht wird, während sie von zwei Breitenseiten der zweiten Materialspule (3f) zwischen einem zweiten Positionierungsabschnitt (25fa) und einem zweiten Verformungsunterdrückungselement eingefügt ist,
der zweite Positionierungsabschnitt (25fa) an einer vorbestimmten Position in einer axialen Richtung der zweiten Drehwelle bereitgestellt ist,
das zweite Verformungsunterdrückungselement ein Element zum Unterdrücken von Breitenverformung der zweiten Materialspule (3f) ist,
wobei
das erste Verformungsunterdrückungselement eine erste Druckplatte (25pb) ist, die eine vorbestimmte Länge in einer Durchmesserrichtung der ersten Drehwelle aufweist, und
die vorbestimmte Länge der ersten Druckplatte (25pb) größer als 1/3 eines Radius der ersten Materialspule (3p) ist, bevor sie als das erste Material (3ps) zugeführt wird, und
wobei
das zweite Verformungsunterdrückungselement eine zweite Druckplatte (25fb) ist, die eine vorbestimmte Länge in einer Durchmesserrichtung der zweiten Drehwelle aufweist, und
die vorbestimmte Länge der zweiten Druckplatte (25fb) größer als 1/3 eines Radius der zweiten Materialspule (3f) ist, bevor sie als das zweite Material (3fs) zugeführt wird.

2. Verfahren zum Herstellen eines saugfähigen Artikels nach Anspruch 1, wobei
das erste Verformungsunterdrückungselement eine erste Druckplatte (25pb) ist, die eine vorbestimmte Länge in einer Durchmesserrichtung der ersten Drehwelle aufweist, und
die vorbestimmte Länge der ersten Druckplatte (25pb) kleiner als ein Radius der ersten Materialspule (3p) ist, bevor sie als das erste Material (3ps) zugeführt wird, erste Materialspule (3p).

3. Verfahren zum Herstellen eines saugfähigen Artikels nach einem der Ansprüche 1 bis 2, wobei
das zweite Verformungsunterdrückungselement eine zweite Druckplatte (25fb) ist, die eine vorbestimmte Länge in einer Durchmesserrichtung der zweiten Drehwelle aufweist, und
die vorbestimmte Länge der zweiten Druckplatte (25fb) kleiner als ein Radius der zweiten Materialspule (3f) ist, bevor sie als das zweite Material (3fs) zugeführt wird, zweite Materialspule (3f).

4. Verfahren zum Herstellen eines saugfähigen Artikels nach einem der Ansprüche 1 bis 3, wobei
der erste Positionierungsabschnitt (25pa) plattenförmig ist und eine vorbestimmte Länge in einer Durchmesserrichtung der ersten Drehwelle aufweist, und
die vorbestimmte Länge des ersten Positionierungsabschnittes (25pa) kleiner als ein Radius der ersten Materialspule (3p) ist, bevor sie als das erste Material (3ps) zugeführt wird.

5. Verfahren zum Herstellen eines saugfähigen Artikels nach einem der Ansprüche 1 bis 4, wobei
der zweite Positionierungsabschnitt (25fa) plattenförmig ist und eine vorbestimmte Länge in einer Durchmesserrichtung der zweiten Drehwelle aufweist, und
die vorbestimmte Länge des zweiten Positionierungsabschnittes (25fa) kleiner als ein Radius der zweiten Materialspule (3f) ist, bevor sie als das zweite Material (3fs) zugeführt wird.

6. Verfahren zum Herstellen eines saugfähigen Artikels nach einem der Ansprüche 1 bis 5, wobei
das Verfahren ferner einen Korrekturschritt zum Korrigieren einer Position der Außenumfangsfläche der zweiten Materialspule (3f) in einer axialen Richtung der zweiten Drehwelle umfasst,
das Korrigieren durchgeführt wird, sodass eine Zufuhrposition des ersten Materials (3ps) in einer axialen Richtung der ersten Drehwelle mit der Position der Außenumfangsfläche der zweiten Materialspule (3f) in der axialen Richtung der zweiten Drehwelle übereinstimmt,
das Korrigieren durch eine Korrekturvorrichtung durchgeführt wird, die konfiguriert ist, um Korrektur durchzuführen, indem sie in Kontakt mit einer Seitenfläche eines Außenumfangsabschnittes der zweiten Materialspule (3f) kommt, die an der zweiten Drehwelle angebracht ist, und den Außenumfangsabschnitt von einer Seite zu einer anderen Seite in der axialen Richtung der zweiten Drehwelle bewegt.

7. Verfahren zum Herstellrn eines saugfähigen Artikels nach Anspruch 6, wobei mindestens eine von der Korrekturvorrichtung für die zweite Drehwelle bereitgestellt ist,
die Korrekturvorrichtung einen Bewegungsabschnitt beinhaltet, der konfiguriert ist, um sich entlang einer Durchmesserrichtung der zweiten Drehwelle zu bewegen,
in dem Schritt des Anbringens der zweiten Materialspule
die Korrekturvorrichtung in eine zurückgezogene Position zurückgezogen wird, um nicht in Kontakt mit der zweiten Materialspule (3f) zu kommen, die anzubringen ist, die zurückgezogene Position eine Position ist, die in der Durchmesserrichtung in Bezug auf die Außenumfangsfläche der zweiten Materialspule (3f), die anzubringen ist, außen ist, und
in dem Korrekturschritt
die Korrekturvorrichtung in der Durchmesserrichtung der zweiten Drehwelle von der zurückgezogenen Position nach innen bewegt wird, um in Kontakt mit der Seitenfläche des Außenumfangsabschnittes der zweiten Materialspule (3f) zu kommen.

8. Verfahren zum Herstellen eines saugfähigen Artikels nach Anspruch 6 oder 7, wobei
ein Kontaktabschnitt der Korrekturvorrichtung, der in Kontakt mit der zweiten Materialspule (3f) kommt, durch einen Drehkörper gebildet ist, der sich um eine Drehachse drehen kann, die sich entlang einer Durchmesserrichtung der zweiten Drehwelle erstreckt.

9. Verfahren zum Herstellen eines saugfähigen Artikels nach Anspruch 8, wobei der Drehkörper eine verjüngte Form aufweist, gemäß der ein Durchmesser des Drehkörpers von einer Innenseite zu einer Außenseite in der Durchmesserrichtung der zweiten Drehwelle zunimmt.

10. Verfahren zum Herstellen eines saugfähigen Artikels nach einem der Ansprüche 7 bis 9, wobei
in dem Korrekturschritt
die Korrekturvorrichtung Korrektur über einen gesamten Umfang des Außenumfangsabschnittes der zweiten Materialspule (3f) unter Verwendung von mindestens einem von Folgendem durchführt:
einem Verfahren, in dem die zweite Materialspule (3f) in einem Zustand, in dem die Korrekturvorrichtung an einer vorbestimmten Position in einer Umfangsrichtung der zweiten Materialspule (3f) befestigt ist, um die zweite Drehwelle gedreht wird, und
einem Verfahren, in dem ein Drehbewegungsabschnitt für Bewegung der Korrekturvorrichtung entlang der Umfangsrichtung der zweiten Materialspule (3f) bereitgestellt ist, die um die zweite Drehwelle zentriert ist, und in dem die Korrekturvorrichtung entlang der Umfangsrichtung der zweiten Materialspule (3f) durch den Drehbewegungsabschnitt in einem Zustand, in dem Drehung der zweiten Materialspule (3f) vorübergehend gestoppt ist, bewegt wird.

11. Verfahren zum Herstellen eines saugfähigen Artikels nach einem der Ansprüche 6 bis 10, wobei
das Verfahren ferner einen Erfassungsschritt zum Erfassen der Position der Außenumfangsfläche der zweiten Materialspule (3f) in der axialen Richtung der zweiten Drehwelle mit Verwendung eines Erfassungsabschnittes umfasst, und
in dem Korrekturschritt
eine Korrekturrichtung und eine Korrekturmenge der Korrekturvorrichtung basierend auf Informationen gesteuert werden, die eine Menge an Abweichung zwischen Folgendem angeben:
der Zufuhrposition des ersten Materials (3ps) in der axialen Richtung der ersten Drehwelle, und
der Position der Außenumfangsfläche der zweiten Materialspule (3f) in der axialen Richtung der zweiten Drehwelle, wobei die Position in dem Erfassungsschritt erfasst worden ist.

12. Verfahren zum Herstellen eines saugfähigen Artikels nach einem der Ansprüche 1 bis 11, wobei
sich ein Wicklungsendabschnitt des zweiten Materials (3fs) an der Außenumfangsfläche der zweiten Materialspule (3f) befindet, und
der Wicklungsendabschnitt an der zweiten Materialspule (3f) unter Verwendung eines Verbindungselements befestigt ist, um daran gehindert zu werden, sich von der zweiten Materialspule (3f) zu trennen.

13. Verfahren zum Herstellen eines saugfähigen Artikels nach einem der Ansprüche 1 bis 12, wobei
in dem Schritt des Anbringens der ersten Materialspule
ein Anbringungsroboter die erste Materialspule (3p) an der ersten Drehwelle anbringt, um gegen den ersten Positionierungsabschnitt (25pa) anzustoßen, und
dann nach dem Anbringen der Anbringungsroboter das erste Verformungsunterdrückungselement an der ersten Drehwelle anbringt, sodass die erste Materialspule (3p) von zwei Breitenseiten der ersten Materialspule (3p) durch den ersten Positionierungsabschnitt (25pa) und das erste Verformungsunterdrückungselement eingefügt ist, und
in dem Schritt des Anbringens der zweiten Materialspule
der Anbringungsroboter die zweite Materialspule (3f) an der zweiten Drehwelle anbringt, um gegen den zweiten Positionierungsabschnitt (25fa) anzustoßen, und dann nach dem Anbringen der Anbringungsroboter das zweite Verformungsunterdrückungselement an der zweiten Drehwelle anbringt, sodass die zweite Materialspule (3f) von zwei Breitenseiten der zweiten Materialspule (3f) durch den zweiten Positionierungsabschnitt (25fa) und das zweite Verformungsunterdrückungselement eingefügt ist.

14. Vorrichtung zum Herstellen eines saugfähigen Artikels, umfassend:
eine erste Materialspule (3p), um die ein erstes Material (3ps) gewickelt ist,
wobei eine Größe der ersten Materialspule (3p) in einer Durchmesserrichtung größer als eine Größe der ersten Materialspule (3p) in einer Breitenrichtung ist,
eine zweite Materialspule (3f), um die ein zweites Material (3fs) gewickelt ist,
wobei eine Größe der zweiten Materialspule (3f) in einer Durchmesserrichtung größer als eine Größe der zweiten Materialspule (3f) in einer Breitenrichtung ist,
wobei ohne Unterbrechung der Zufuhr des ersten Materials (3ps), das kontinuierlich von der vorangehenden ersten Materialspule (3p) zugeführt worden ist, das zweite Material (3fs), das von der folgenden zweiten Materialspule (3f) zugeführt wird, mit dem ersten Material (3ps) verbunden wird und dann zugeführt wird;
eine erste Drehwelle, an der eine erste Materialspule (3p) anzubringen ist;
einen Verbindungsabschnitt, der konfiguriert ist, um das zweite Material (3fs) mit dem ersten Material (3ps) zu verbinden, indem durch angetriebene Drehung der ersten Drehwelle das erste Material (3ps), das von der ersten Materialspule (3p) zugeführt wird, gegen eine Außenumfangsfläche der zweiten Materialspule (3f) gedrückt wird, die sich aufgrund von angetriebener Drehung der zweiten Drehwelle dreht,
einen ersten Positionierungsabschnitt (25pa), der an einer vorbestimmten Position in einer axialen Richtung der ersten Drehwelle bereitgestellt ist,
ein erstes Verformungsunterdrückungselement, das konfiguriert ist, um Breitenverformung der ersten Materialspule (3p) zu unterdrücken,
wobei der erste Positionierungsabschnitt (25pa) und das erste Verformungsunterdrückungselement bereitgestellt sind, um die erste Materialspule (3p) von zwei Breitenseiten der ersten Materialspule (3p) während Anbringung einzufügen;
einen zweiten Positionierungsabschnitt (25fa), der an einer vorbestimmten Position in einer axialen Richtung der zweiten Drehwelle bereitgestellt ist; und
ein zweites Verformungsunterdrückungselement, das konfiguriert ist, um Breitenverformung der zweiten Materialspule (3f) zu unterdrücken,
wobei der zweite Positionierungsabschnitt (25fa) und das zweite Verformungsunterdrückungselement bereitgestellt sind, um die zweite Materialspule (3f) von zwei Breitenseiten der zweiten Materialspule (3f) während Anbringung einzufügen
wobei
das erste Verformungsunterdrückungselement eine erste Druckplatte (25pb) ist, die eine vorbestimmte Länge in einer Durchmesserrichtung der ersten Drehwelle aufweist, und
die vorbestimmte Länge der ersten Druckplatte (25pb) größer als 1/3 eines Radius der ersten Materialspule (3p) ist, bevor sie als das erste Material (3ps) zugeführt wird, und
wobei
das zweite Verformungsunterdrückungselement eine zweite Druckplatte (25fb) ist, die eine vorbestimmte Länge in einer Durchmesserrichtung der zweiten Drehwelle aufweist, und
die vorbestimmte Länge der zweiten Druckplatte (25fb) größer als 1/3 eines Radius der zweiten Materialspule (3f) ist, bevor sie als das zweite Material (3fs) zugeführt wird.

## Revendications

1. Procédé de fabrication d'un article absorbant dans lequel, sans arrêter l'alimentation d'un premier matériau (3ps) qui a été alimenté en continu à partir d'une bobine de premier matériau (3p) précédente, un second matériau (3fs) fourni à partir d'une bobine de second matériau (3f) suivante est connecté au premier matériau (3ps), puis l'alimentation du second matériau (3fs),
le procédé de fabrication de l'article absorbant comprenant :
une étape de fixation de bobine de premier matériau consistant à fixer la bobine de premier matériau (3p) à un premier arbre de rotation, le premier matériau étant enroulé autour de la bobine de premier matériau (3p) ;
une étape de fixation de bobine de second matériau consistant à fixer une bobine de second matériau (3f) à un second arbre de rotation, le second matériau (3fs) étant enroulé autour de la bobine de second matériau (3f) ; et
une étape de connexion consistant à connecter le second matériau (3fs) au premier matériau (3ps) en pressant le premier matériau (3ps) contre une surface circonférentielle externe de la bobine de second matériau (3f),
le premier matériau (3ps) étant alimenté à partir de la bobine de premier matériau (3p) en raison de la rotation entraînée du premier arbre de rotation,
la bobine de second matériau (3f) tournant en raison de la rotation entraînée du second arbre de rotation,
une taille de la bobine de premier matériau (3p) dans une direction de diamètre étant supérieure à une taille de la bobine de premier matériau (3p) dans une direction de largeur,
une taille de la bobine de second matériau (3f) dans une direction de diamètre étant supérieure à une taille de la bobine de second matériau (3f) dans une direction de largeur,
dans l'étape de fixation de bobine de premier matériau,
la bobine de premier matériau (3p) étant fixée tout en étant prise en sandwich à partir de deux côtés dans le sens de la largeur de la bobine de premier matériau (3p) entre une première partie de positionnement (25pa) et un premier élément de suppression de déformation,
la première partie de positionnement (25pa) étant prévue à une position prédéterminée dans une direction axiale du premier arbre de rotation,
le premier élément de suppression de déformation étant un élément pour supprimer la déformation dans le sens de la largeur de la bobine de premier matériau (3p),
dans l'étape de fixation de bobine de second matériau,
la bobine de second matériau (3f) étant fixée tout en étant prise en sandwich à partir de deux côtés dans le sens de la largeur de la bobine de second matériau (3f) entre une seconde partie de positionnement (25fa) et un second élément de suppression de déformation,
la seconde partie de positionnement (25fa) étant prévue à une position prédéterminée dans une direction axiale du second arbre de rotation,
le second élément de suppression de déformation étant un élément pour supprimer la déformation dans le sens de la largeur de la bobine de second matériau (3f),
dans lequel
le premier élément de suppression de déformation est une première plaque de pression (25pb) qui a une longueur prédéterminée dans une direction de diamètre du premier arbre de rotation, et
la longueur prédéterminée de la première plaque de pression (25pb) est supérieure à 1/3 d'un rayon de la bobine de premier matériau (3p) avant d'être alimentée en tant que premier matériau (3ps), et dans lequel
le second élément de suppression de déformation est une seconde plaque de pression (25fb) qui a une longueur prédéterminée dans une direction de diamètre du second arbre de rotation, et
la longueur prédéterminée de la seconde plaque de pression (25fb) est supérieure à 1/3 d'un rayon de la bobine de second matériau (3f) avant d'être alimentée en tant que second matériau (3fs).

2. Procédé de fabrication d'un article absorbant selon la revendication 1, dans lequel
le premier élément de suppression de déformation est une première plaque de pression (25pb) qui a une longueur prédéterminée dans une direction de diamètre du premier arbre de rotation, et
la longueur prédéterminée de la première plaque de pression (25pb) est inférieure à un rayon de la bobine de premier matériau (3p) avant d'être alimentée en tant que premier matériau (3ps), bobine de premier matériau (3p).

3. Procédé de fabrication d'un article absorbant selon l'une quelconque des revendications 1 et 2, dans lequel
le second élément de suppression de déformation est une seconde plaque de pression (25fb) qui a une longueur prédéterminée dans une direction de diamètre du second arbre de rotation, et
la longueur prédéterminée de la seconde plaque de pression (25fb) est inférieure à un rayon de la bobine de second matériau (3f) avant d'être alimentée en tant que second matériau (3fs). bobine de second matériau (3f).

4. Procédé de fabrication d'un article absorbant selon l'une quelconque des revendications 1 à 3, dans lequel
la première partie de positionnement (25pa) est en forme de plaque et a une longueur prédéterminée dans une direction de diamètre du premier arbre de rotation, et
la longueur prédéterminée de la première partie de positionnement (25pa) est inférieure à un rayon de la bobine de premier matériau (3p) avant d'être alimentée en tant que premier matériau (3ps).

5. Procédé de fabrication d'un article absorbant selon l'une quelconque des revendications 1 à 4, dans lequel
la seconde partie de positionnement (25fa) est en forme de plaque et a une longueur prédéterminée dans une direction de diamètre du second arbre de rotation, et
la longueur prédéterminée de la seconde partie de positionnement (25fa) est inférieure à un rayon de la bobine de second matériau (3f) avant d'être alimentée en tant que second matériau (3fs).

6. Procédé de fabrication d'un article absorbant selon l'une quelconque des revendications 1 à 5, dans lequel
le procédé comprend en outre une étape de correction consistant à corriger une position de la surface circonférentielle externe de la bobine de second matériau (3f) dans une direction axiale du second arbre de rotation,
la correction étant effectuée de sorte qu'une position d'alimentation du premier matériau (3ps) dans une direction axiale du premier arbre de rotation correspond à la position de la surface circonférentielle externe de la bobine de second matériau (3f) dans la direction axiale du second arbre de rotation,
la correction étant effectuée par un dispositif de correction configuré pour effectuer une correction en venant en contact avec une surface latérale d'une partie circonférentielle externe de la bobine de second matériau (3f) fixée au second arbre de rotation et en déplaçant la partie circonférentielle externe d'un côté vers un autre côté dans la direction axiale du second arbre de rotation.

7. Procédé de fabrication d'un article absorbant selon la revendication 6, dans lequel
au moins l'un du dispositif de correction est prévu pour le second arbre de rotation, le dispositif de correction comporte une partie mobile configurée pour se déplacer le long d'une direction de diamètre du second arbre de rotation,
dans l'étape de fixation de bobine de second matériau,
le dispositif de correction est rétracté dans une position rétractée pour ne pas entrer en contact avec la bobine de second matériau (3f) qui doit être fixée,
la position rétractée étant une position qui est vers l'extérieur dans la direction de diamètre par rapport à la surface circonférentielle externe de la bobine de second matériau (3f) qui doit être fixée, et
dans l'étape de correction,
le dispositif de correction est déplacé vers l'intérieur dans la direction de diamètre du second arbre de rotation à partir de la position rétractée de manière à entrer en contact avec la surface latérale de la partie circonférentielle externe de la bobine de second matériau (3f).

8. Procédé de fabrication d'un article absorbant selon la revendication 6 ou 7, dans lequel
une partie de contact du dispositif de correction qui vient en contact avec la bobine de second matériau (3f) est constituée par un corps rotatif qui peut tourner autour d'un axe de rotation s'étendant selon une direction de diamètre du second arbre de rotation.

9. Procédé de fabrication d'un article absorbant selon la revendication 8, dans lequel
le corps rotatif a une forme conique selon laquelle un diamètre du corps rotatif augmente d'un côté intérieur vers un côté extérieur dans la direction de diamètre du second arbre de rotation.

10. Procédé de fabrication d'un article absorbant selon l'une quelconque des revendications 7 à 9, dans lequel
dans l'étape de correction,
le dispositif de correction effectue une correction sur toute une circonférence de la partie circonférentielle externe de la bobine de second matériau (3f) à l'aide d'au moins l'un parmi
un procédé dans lequel la bobine de second matériau (3f) est mise en rotation autour du second arbre de rotation dans un état dans lequel le dispositif de correction est fixé à une position prédéterminée dans une direction circonférentielle de la bobine de second matériau (3f), et
un procédé dans lequel une partie mobile de rotation est prévue pour le déplacement du dispositif de correction le long de la direction circonférentielle de la bobine de second matériau (3f) centrée autour du second arbre de rotation, et dans lequel le dispositif de correction est déplacé le long de la direction circonférentielle de la bobine de second matériau (3f) par la partie mobile de rotation dans un état dans lequel la rotation de la bobine de second matériau (3f) est temporairement arrêtée.

11. Procédé de fabrication d'un article absorbant selon l'une quelconque des revendications 6 à 10, dans lequel
le procédé comprend en outre une étape de détection consistant à détecter la position de la surface circonférentielle externe de la bobine de second matériau (3f) dans la direction axiale du second arbre de rotation à l'aide d'une partie de détection, et dans l'étape de correction,
une direction de correction et une quantité de correction du dispositif de correction sont commandées sur la base d'informations indiquant une quantité d'écart entre la position d'alimentation du premier matériau (3ps) dans la direction axiale du premier arbre de rotation et
la position de la surface circonférentielle externe de la bobine de second matériau (3f) dans la direction axiale du second arbre de rotation, la position ayant été détectée lors de l'étape de détection.

12. Procédé de fabrication d'un article absorbant selon l'une quelconque des revendications 1 à 11, dans lequel
une partie d'extrémité d'enroulement du second matériau (3fs) est située au niveau de la surface circonférentielle externe de la bobine de second matériau (3f), et
la partie d'extrémité d'enroulement est fixée à la bobine de second matériau (3f) à l'aide d'un élément de jonction de manière à être empêchée de se séparer de la bobine de second matériau (3f).

13. Procédé de fabrication d'un article absorbant selon l'une quelconque des revendications 1 à 12, dans lequel
dans l'étape de fixation de bobine de premier matériau,
un robot de fixation fixe la bobine de premier matériau (3p) au premier arbre de rotation de manière à venir en butée contre la première partie de positionnement (25pa), et
ensuite, après la fixation, le robot de fixation fixe le premier élément de suppression de déformation au premier arbre de rotation de sorte que la bobine de premier matériau (3p) est prise en sandwich à partir de deux côtés dans le sens de la largeur de la bobine de premier matériau (3p) par la première partie de positionnement (25pa) et le premier élément de suppression de déformation, et
dans l'étape de fixation de bobine de second matériau,
le robot de fixation fixe la bobine de second matériau (3f) au second arbre de rotation de manière à venir en butée contre la seconde partie de positionnement (25fa), et ensuite, après la fixation, le robot de fixation fixe le second élément de suppression de déformation au second arbre de rotation de sorte que la bobine de second matériau (3f) est prise en sandwich à partir de deux côtés dans le sens de la largeur de la bobine de second matériau (3f) par la seconde partie de positionnement (25fa) et le second élément de suppression de déformation.

14. Dispositif de fabrication d'un article absorbant, comprenant :
une bobine de premier matériau (3p) autour de laquelle un premier matériau (3ps) est enroulé,
une taille de la bobine de premier matériau (3p) dans une direction de diamètre étant supérieure à une taille de la bobine de premier matériau (3p) dans une direction de largeur ;
une bobine de second matériau (3f) autour de laquelle un second matériau (3fs) est enroulé,
une taille de la bobine de second matériau (3f) dans une direction de diamètre étant supérieure à une taille de la bobine de second matériau (3f) dans une direction de largeur,
sans arrêter l'alimentation du premier matériau (3ps) qui a été alimenté en continu à partir de la bobine de premier matériau (3p) précédente, le second matériau (3fs) alimenté à partir de la bobine de second matériau (3f) suivante étant joint au premier matériau (3ps) puis étant alimenté ;
un premier arbre de rotation auquel une bobine de premier matériau (3p) doit être fixée ;
une partie de connexion configurée pour joindre le second matériau (3fs) au premier matériau (3ps) en pressant, par la rotation entraînée du premier arbre de rotation, le premier matériau (3ps) alimenté à partir de la bobine de premier matériau (3p) contre une surface circonférentielle externe de la bobine de second matériau (3f) qui tourne en raison de la rotation entraînée du second arbre de rotation,
une première partie de positionnement (25pa) qui est prévue à une position prédéterminée dans une direction axiale du premier arbre de rotation ;
un premier élément de suppression de déformation configuré pour supprimer la déformation dans le sens de la largeur de la bobine de premier matériau (3p),
la première partie de positionnement (25pa) et le premier élément de suppression de déformation étant prévus pour prendre en sandwich la bobine de premier matériau (3p) à partir de deux côtés dans le sens de la largeur de la bobine de premier matériau (3p) pendant la fixation ;
la seconde partie de positionnement (25fa) qui est prévue à une position prédéterminée dans une direction axiale du second arbre de rotation ; et
un second élément de suppression de déformation configuré pour supprimer la déformation dans le sens de la largeur de la bobine de second matériau (3f),
la seconde partie de positionnement (25fa) et le second élément de suppression de déformation étant prévus pour prendre en sandwich la bobine de second matériau (3f) à partir de deux côtés dans le sens de la largeur de la bobine de second matériau (3f) pendant la fixation,
dans lequel
le premier élément de suppression de déformation est une première plaque de pression (25pb) qui a une longueur prédéterminée dans une direction de diamètre du premier arbre de rotation, et
la longueur prédéterminée de la première plaque de pression (25pb) est supérieure à 1/3 d'un rayon de la bobine de premier matériau (3p) avant d'être alimentée en tant que premier matériau (3ps), et dans lequel
le second élément de suppression de déformation est une seconde plaque de pression (25fb) qui a une longueur prédéterminée dans une direction de diamètre du second arbre de rotation, et
la longueur prédéterminée de la seconde plaque de pression (25fb) est supérieure à 1/3 d'un rayon de la bobine de second matériau (3f) avant d'être alimentée en tant que second matériau (3fs).
